# EUROPÄISCHE PATENTSCHRIFT

(11) **EP 3 836 901 B1**
(45) Veröffentlichungstag und Bekanntmachung des Hinweises auf die Patenterteilung: **15.02.2023**
(21) Anmeldenummer: 19725026.9
(22) Anmeldetag: 29.04.2019
(51) Int. Cl.: A61K 9/127, A61K 36/185

(54) **VERFAHREN ZUR VERKAPSELUNG VON WIRKSTOFFEN IN LIPOSOMEN**
METHOD FOR ENCAPSULATING ACTIVE SUBSTANCES AGENTS IN LIPOSOMES
PROCÉDÉ POUR ENCAPSULER DES PRINCIPES ACTIFS DANS DES LIPOSOMES

(30) Priorität: 14.08.2018 DE 102018006439
(43) Veröffentlichungstag der Anmeldung: 23.06.2021
(73) Patentinhaber: ABNOBA GmbH, 75223 Niefern-Öschelbronn (DE)
(72) Erfinder: LENEWEIT, Gero, 75223 Niefern-Öschelbronn (DE); SANTOS DE MIRANDA, Bàrbara, 75177 Pforzheim (DE)
(74) Vertreter: Lichti - Patentanwälte Partnerschaft mbB
(86) Internationale Anmeldenummer: PCT/EP2019/000132
(87) Internationale Veröffentlichungsnummer: WO 2020/035161

(56) Entgegenhaltungen:
- Ellen Hildebrandt ET AL: "Liposomal formulations of mistletoe produced by centrifugal technologies and cell proliferation analysis of both mistletoe extracts and isolated mistletoe lectin I = Liposomale Formulierung von Mistelextrakten durch Zentrifugationsverfahren und Analyse der Zellproliferation von Gesamtextrakten und i", , 1. Januar 2016 (2016-01-01), XP55605767, DOI: 10.5445/IR/1000063594 Gefunden im Internet: URL:https://pdfs.semanticscholar.org/e58c/ faff65f3e6ba81ec15bb11447072fc02114b.pdf?_ ga=2.230300312.583365019.1563266730-787531 10.1549445781
- YOGITA P. PATIL ET AL: "Novel methods for liposome preparation", CHEMISTRY AND PHYSICS OF LIPIDS., Bd. 177, 9. November 2013 (2013-11-09), Seiten 8-18, XP55605974, IR ISSN: 0009-3084, DOI: 10.1016/j.chemphyslip.2013.10.011
- MASSING ET AL: "Dual asymmetric centrifugation (DAC)-A new technique for liposome preparation", JOURNAL OF CONTROLLED RELEASE, ELSEVIER, AMSTERDAM, NL, Bd. 125, Nr. 1, 5. Dezember 2007 (2007-12-05), Seiten 16-24, XP022375059, ISSN: 0168-3659, DOI: 10.1016/J.JCONREL.2007.09.010

## Beschreibung

Die Erfindung betrifft ein Verfahren zur Verkapselung von Wirkstoffen in Liposomen, umfassend
- eine, insbesondere hydrophile, Lösung des Wirkstoffes und
- wenigstens eine Bischicht aus zwei zumindest monomolekularen Schichten aus wenigstens einer ersten amphiphilen Verbindung und wenigstens einer zweiten amphiphilen Verbindung, insbesondere jeweils aus der Gruppe der Lipide,
wobei die Wirkstofflösung durch die Bischicht verkapselt ist.

Unter Liposomen, welche auch als Vesikel bezeichnet werden, versteht man Membranbläschen, welche in der Regel in einem hydrophilen, insbesondere wässrigen, Milieu kolloidal suspendiert vorliegen können und eine flüssige Phase einschließen, wobei es sich bei der flüssigen Phase üblicherweise, wenn auch nicht notwendigerweise, um eine hydrophile, zumeist wässrige Phase handelt. Die Membranhülle, welche die flüssige Phase einschließt, ist aus einer Doppel- bzw. Bischicht aus zwei zumindest monomolekularen Schichten entweder aus je ein und derselben oder auch aus verschiedenen Molekülen gebildet, welche jeweils sowohl einen unpolaren, also hydrophoben bzw. lipophilen, Teil als auch einen polaren, also hydrophilen bzw. lipophoben, Teil aufweisen und aufgrund dieser Eigenschaften als amphiphil bezeichnet werden. Bei den hierbei als membranbildende Moleküle zum Einsatz gelangenden amphiphilen Verbindungen handelt es sich zumeist um Lipide, wie beispielsweise um Phospholipide, Sphingolipide, Glycolipide, Fettsäuren oder dergleichen, wobei jedoch auch andere amphiphile Verbindungen als Membrankomponenten verwendet werden können, wie beispielsweise Lipopolysaccharide, Tocopherole, Squalene, Sterine bzw. Sterole, Cholesterole etc. Anlässlich der Erzeugung der Bischicht von Liposomen ordnen sich die amphiphilen Verbindungen entsprechend ihrer hydrophilen/hydrophoben Eigenschaften an, so dass die hydrophoben Teile der amphiphilen Verbindungen jeweils zueinander gerichtet, um einen möglichst geringen Kontakt zu der zu verkapselnden, z.B. hydrophilen bzw. wässrigen Phase, haben, wohingegen die hydrophilen Teile der amphiphilen Verbindungen zu der z.B. hydrophilen bzw. wässrigen Phase innerhalb und außerhalb des Liposoms gerichtet sind. Aufgrund des thermodynamischen Bestrebens, bei einer solchen Ausrichtung eine energetisch günstige Form mit einer möglichst geringen Oberfläche einzunehmen, weisen Liposomen üblicherweise eine im Wesentlichen kugelförmige bzw. sphärische Form auf. Die amphiphilen Verbindungen, welche die Bischicht(en) der Membran von Liposomen bilden, halten folglich lediglich durch nicht kovalente Bindungskräfte zusammen, weshalb die Membran einen vornehmlich fluiden Charakter besitzt.

In Ergänzung zur üblichen wissenschaftlichen Verwendung der Bezeichnung "Liposom" oder des hiermit synonymen Begriffs "Vesikel" umfassen Liposomen im Rahmen der vorliegenden Offenbarung auch kolloidchemischen Aggregate in Form von Nanokapseln aus jeglichen amphiphilen Substanzen, Polymerliposomen, Lipidnanopartikel und Mischungen solcher Aggregatbildungen mit reinen Liposomen. Daher werden derartige kolloidchemische Aggregate im Rahmen der vorliegenden Offenbarung stets mit einbezogen, auch wenn nur der Begriff "Liposom" verwendet wird.

Die Größe und die genaue Form von Liposomen hängen maßgeblich vom chemischen Aufbau der die Bischicht(en) ihrer Membran bildenden amphiphilen Verbindung(en) ab sowie von den physikalisch-chemischen Eigenschaften der zu verkapselnden, üblicherweise hydrophilen bzw. wässrigen Phase, wie z.B. deren Ionenstärke, pH-Wert, Osmolalität und dergleichen, und dem jeweiligen Herstellungsverfahren. Liposomen können dabei einerseits nur eine einzige Bischicht besitzen, wobei solche Liposomen als unilamellar ("unilamellare Vesikel", ULV) bezeichnet werden, oder sie können mehrere, jeweils konzentrisch angeordnete Bischichten aufweisen, wobei solche Liposomen als multilamellar ("multilamellare Vesikel", MLV) bezeichnet werden. Der mittlere Durchmesser beträgt dabei in der Regel zwischen etwa 20 nm und etwa 100 um, insbesondere zwischen etwa 25 nm und etwa 30 um.

Wie bereits erwähnt, können die die Bischicht(en) der Membran von Liposomen bildenden amphiphilen Verbindungen je nach angestrebtem pharmakodynamischem Wirkprofil, pharmakokinetischem Verhalten, chemischen und physikalischen Eigenschaften, wie beispielsweise Größe, Größenverteilung, Lamellarität, Fluidität, Permeabilität, Zetapotential, Phasenübergangstemperatur der Membran etc., aus denselben oder aus unterschiedlichen Molekülen, z.B. aus der Gruppe der Lipide, gebildet sein, wobei die Bischicht einerseits aus derselben amphiphilen Verbindung oder aus denselben Mischungen mehrerer amphiphilen Verbindungen aufgebaut sein kann, oder die einzelnen, zumindest monomolekularen Schichten der Bischicht können jeweils aus unterschiedlichen amphiphilen Verbindungen oder aus Mischungen von unterschiedlichen amphiphilen Verbindungen aufgebaut sein. Während man im erstgenannten Fall von symmetrischen Liposomen spricht (die zumindest monomolekularen Schichten der Bischicht der Membran sind von demselben Aufbau, wobei die Moleküle der Schichten hinsichtlich ihres polaren und unpolaren Teils aber entgegengesetzt zueinander ausgerichtet sind), spricht man im letztgenannten Fall von asymmetrischen Liposomen (die zumindest monomolekularen Schichten der Bischicht der Membran sind von unterschiedlichem Aufbau, wobei die Moleküle der Schichten hinsichtlich ihres polaren und unpolaren Teils wiederum entgegengesetzt zueinander ausgerichtet sind).

Während Liposomen beispielsweise auch für Untersuchungen der biophysikalischen Eigenschaften von Biomembranen Verwendung finden können, kommen sie vornehmlich im kosmetischen sowie insbesondere im medizinischen Bereich zum Einsatz. Dabei ist es insbesondere möglich, durch die liposomale Formulierung von Wirkstoffen, wie Arzneimitteln und dergleichen, empfindliche Wirkstoffe nach der Applikation vor einer möglichen Metabolisierung zu schützen und gezielt an die Zellen des Organismus' heranzuführen, wo der Wirkstoff seine Wirkung entfalten soll, so dass etwaige Nebenwirkungen des liposomal formulierten Wirkstoffes verringert und die Wirksamkeit erhöht werden kann, um niedrigere Dosen des Wirkstoffes verabreichen zu können. Darüber hinaus kann durch die Verkapselung von Wirkstoffen in Liposomen die Plasmahalbwertszeit erhöht werden. Der Wirkstoff liegt hierbei in dem Liposom üblicherweise in Form einer zumeist hydrophilen, insbesondere wässrigen oder beispielsweise auch alkoholischen, Wirkstofflösung vor. Vornehmlich hydrophile Wirkstoffe können dabei mehr oder minder vollständig in die Liposomen eingekapselt sein, während vornehmlich lipophile Stoffe eher in die Bischicht der amphiphilen Verbindung(en) eingebaut werden.

Demzufolge spielen Liposomen in der modernen Pharmazie, Kosmetik und Lebensmitteltechnologie eine bedeutende Rolle als Transportvehikel für pharmazeutische oder andersartige Wirkstoffe, zur Verbesserung der Hautfeuchtigkeit oder Wirkstoffaufnahme bzw. für hochwertige Lebensmittelzusätze. Weitere, lediglich exemplarisch genannte Einsatzgebiete von Liposomen umfassen die gezielte Arzneistoff-Zuführung ("Drug Delivery"), die synthetische Chemie allgemein, nanoskalige Reaktionskammern sowie allgemeine technologische Entwicklungen in den Bereichen der Energie, Optik, Elektronik, Mikrofluidik, Kolloidchemie, Biosensorik oder artverwandte Gebiete, in welchen Liposomen Verwendung finden können.

Liposomen können darüber hinaus mit einer Polymerschicht, z.B. auf der Basis von Polyethylenglykol (PEG), überzogen und/oder kann zumindest die amphiphile Verbindung der äußeren Schicht der die Membran bildenden Bischicht(en) mit einem solchen Polymer modifiziert sein, wobei man in diesem Fall von "PEGylierten Liposomen" spricht. Die Polymerschicht dient dabei zum sterischen Schutz der Membran und sie schützt und verringert eine Markierung (Opsonierung) und Eliminierung durch das Immunsystem, wodurch die Liposomen länger im Organismus zirkulieren und sich z.B. in Tumorgewebe anreichern können. Während, wie oben bereits erwähnt, die Pharmakokinetik von der Natur der Liposomen, insbesondere von den die Bischicht(en) ihrer Membran bildenden amphiphilen Verbindung(en) und kaum mehr von der Natur des verkapselten Wirkstoffes selbst beeinflusst wird, lässt sich das sogenannte Drug Targeting durch eine solche PEGylierung weiter optimieren, was z.B. für die Tumortherapie eine maximale Anreicherung der Wirkstoffe im Tumorgewebe bedeutet. Aus diesem Grunde werden gegenwärtig viele Chemotherapeutika als liposomale Formulierungen appliziert (z.B. DaunoXome^{®}, DepoCyt^{®}, Doxil^{®}/Caelyx^{®} Marqibo^{®}, Mepact^{®}, Myocet^{®}), um durch die Anreicherung im Zielgewebe die therapeutischen Wirkungen zu erhöhen, die Nebenwirkungen durch die verringerte Freisetzung in gesundem Gewebe und Organen aber gleichzeitig zu verringern.

Bisherige kommerzielle Herstellungstechniken von Liposomen beruhen im Wesentlichen auf den folgenden drei alternativen Verfahren:
a) den Homogenisierungsmethoden, bei welchen zunächst Rohliposomen erzeugt und diese anschließend mechanisch auf eine einheitliche Größe gebracht werden;
b) der Detergensdialyse; und
c) der Ethanol-Injektionsmethode.
Den Verfahren unter obiger Ziffer b) und c) ist gemeinsam, dass durch Hilfsstoffe die Löslichkeit der die Bischicht bildenden amphiphilen Verbindungen, wie z.B. Phospholipiden, in wässrigen Lösungen verbessert wird und es durch Abtrennung oder Verdünnung der Hilfsstoffe zur Bildung der Bischichten kommt, welche sich zu geschlossenen Liposomen formen.

Allen bisher entwickelten, kommerziellen Herstellungstechniken für Liposomen ist gemein, dass bei der Einkapselung der üblicherweise hydrophile bzw. wässrige oder gegebenenfalls auch alkoholische Innenraum identisch mit dem Außenraum ist. Bei einer üblichen Einkapselungskapazität von ca. 1 bis 15 % bedeutet dies, dass 99 bis 85% des Wirkstoffs nachträglich aus dem Außenraum entfernt und vorzugsweise wiederverwendet werden müssen. Eine nachträgliche Beladung ("Remote Loading") der Liposomen mittels thermischer Methoden oder pH-Gradienten ist nur für bestimmte Wirkstoffe möglich und bringt andere Nachteile mit sich, wie z.B. verringerte Lagerstabilitat, zusätzliche Bearbeitungsschritte und dergleichen. Darüber hinaus werden eine Vielzahl von biogenen pharmazeutischen Wirkstoffen, insbesondere Proteine, durch diese Methoden denaturiert.

Eine weitere Unzulänglichkeit der bisher entwickelten, kommerziellen Herstellungsverfahren stellt insbesondere die Uniformität der Membran dar, d.h. die Innen- und Außenseite der Bischicht sind mehr oder minder identisch; bei den erzeugten Liposomen handelt es sich folglich um symmetrische Liposomen. Bei allen natürlich vorkommenden, biogenen Membranen sind die Innen- und Außenseiten der Membran jedoch stets unterschiedlich, was sowohl im Hinblick auf ihre Lipid- als auch auf ihre Proteinkomposition gilt. Die Asymmetrie biologischer Membranen besitzt eine wichtige physiologische Funktion für verschiedene zelluläre Erkennungs- und Transportmechanismen. Entsprechend wäre die uneingeschränkte Manipulierbarkeit der Zusammensetzung der Innen- und Außenseite von Liposomenmembranen wünschenswert, z.B. für die Anlagerung von Wirkstoffen auf der Innenseite und von sterischen Schutzschichten oder Rezeptoren auf der Außenseite, was derzeit nur durch nachträgliche Bearbeitungsschritte eingeschränkt möglich ist.

Es sei an dieser Stelle darauf hingewiesen, dass unter dem Begriff "Monoschicht" üblicherweise monomolekulare Schichten von amphiphilen bzw. grenzflächenaktiven Verbindungen, z.B. von Lipiden verstanden werden, wobei sich ein Emulsionstropfen, welcher nur über eine monomolekulare bzw. einfache Schicht aus einer amphiphilen Verbindung oder aus einer Gruppe von amphiphilen Verbindungen verfügt, von Liposomen dadurch unterscheidet, dass letztere wenigstens eine Bischicht aus zwei zumindest monomolekularen Schichten amphiphiler Verbindungen aufweisen, deren polare und unpolare Bereiche in den die Bischicht bildenden Monoschichten jeweils entgegengesetzt zueinander orientiert sind. In Ergänzung zu dieser Definition sind im Rahmen der vorliegenden Offenbarung mit dem Begriff "Monoschicht" stets auch alle Substanzen und Substanzklassen angesprochen, welche in der Lage sind, dünne Schichten zu bilden, wie z. B. Polymere und Proteine, was insbesondere auch dann gilt, wenn diese Substanzklassen und Substanzen gegebenenfalls mehr als eine einzige Molekülschicht umfassen oder umfassen können. Analog ist unter "Bischicht" im Rahmen der vorliegenden Offenbarung stets auch die Zusammenfügung zweier Monoschichten im obigen Sinne zu verstehen. Unter "Pre-Liposomen" sind im Rahmen der vorliegenden Offenbarung Emulsionstropfen zu verstehen, welche über eine monomolekulare bzw. einfache Schicht aus einer amphiphilen Verbindung oder aus einer Gruppe von amphiphilen Verbindungen verfügen.

Die der Erfindung zugrundeliegende Möglichkeit, Monoschichten aus amphiphilen Verbindungen zu einer Bischicht zu synthetisieren und durch Zentrifugalkräfte zu Liposomen abzuschnüren, ist grundsätzlich aus dem Stand der Technik bekannt.

So beschreiben H. Träuble und E. Grell: "The formation of asymmetrical spherical lecithin vesicles", Neuroscience Research Program Bulletin, 9, 373-3801 (1971) ein Verfahren zur Verkapselung von Wirkstoffen in Liposomen, wobei in einem Schritt (a) eine wässrige Wirkstofflösung bereitgestellt wird, welche in einem Schritt (b) in einer hiermit nicht bzw. schlecht mischbaren Phase in Gegenwart von mehreren amphiphilen Verbindungen in Form von Lipiden dispergiert wird, so dass sich zumindest eine der amphiphilen Verbindungen als eine monomolekulare Schicht an die emulgierten Tropfen der Wirkstofflösung anlagert, also Pre-Liposomen im Sinne der vorliegenden Offenbarung bildet. Ferner wird in einem Schritt (c) eine mit der kontinuierlichen Phase der Emulsion nicht bzw. schlecht mischbare flüssige - hier wässrige - Phase bereitgestellt und werden die derart erzeugten Pre-Liposomen durch Zentrifugalkräfte aus der Emulsion in die wässrige Phase überführt. Beim Phasendurchtritt umgeben sich die Pre-Liposomen mit einer zweiten Monoschicht aus wenigstens einer der amphiphilen Verbindungen, welche an der Phasengrenze angereichert worden ist, so dass unter Erhalt einer Bischicht das Liposom bzw. das Vesikel erzeugt wird. Während sich das Verfahren zweifellos zur Erzeugung von symmetrischen Liposomen eignet, wird in dem Aufsatz die Vermutung aufgestellt, dass es hierdurch auch möglich ist, Liposomen mit einer asymmetrischen Zusammensetzung der aus den verschiedenartigen ersten amphiphilen Verbindungen konstituierten Bischicht zu bilden, jedoch keine Nachweise für die Durchführbarkeit dieser Idee geliefert. Das ungelöste Problem dieses Konzeptes besteht nämlich darin, dass die amphiphilen Verbindungen, welche die Phasengrenzen von a) den dispergierten Tropfen und b) der Phasengrenze zwischen den beiden nicht mischbaren Phasen belegen, nicht voneinander getrennt werden können. Aufgrund einer fehlenden Abtrennung können die amphiphilen Verbindungen somit alle Grenzflächen durch Diffusion erreichen, wodurch keine hinreichende Asymmetrie der Bischicht erreicht werden kann. Ein weiteres Problem dieses Konzeptes besteht in der Eigenschaft fast aller amphiphiler Verbindungen, wie z.B. Phospholipiden und dergleichen, gemeinsam mit einer organischen und einer wässrigen Phase sogenannte Organogele zu bilden (vgl. hierzu z.B. auch P. L. Luisi, R. Scartazzini, G. Haering, P. Schurtenberger: "Organogels from water-in-oil microemulsions", Colloid Polym Sci, 268: 356-374 (1990)) Diese Organogele reichern sich an der Phasengrenze zwischen der organischen und der wässrigen Phase an und bilden eine zunehmend schwerer zu überwindende Sperrschicht, welche den Phasendurchtritt behindert.

Dasselbe gilt für ein entsprechendes Verfahren zur Herstellung von Liposomen aus einer Wirkstofflösung auf der Basis von Mistelextrakten und amphiphilen Verbindungen aus der Gruppe der Phospholipide, wie es aus dem Aufsatz "Liposomale Formulierung von Mistelextrakten durch Zentrifugationsverfahren und Analyse der Zellproliferation von Gesamtextrakten und isoliertem Mistellektin I" von E. Hildebrandt, C. Heyder, M. B. C. de Matos, N. Beztsinna, R. J. Kok, H. Nirschl und G. Leneweit (01.01.2016; XP55605767) bekannt ist.

Eine Weiterentwicklung der beiden oben beschriebenen Verfahren von Träuble und Grell bzw. von Hildebrandt et al. stellt das Verfahren gemäß dem Aufsatz von S. Pautot, B. J. Frisken, D. A. Weitz: "Engineering asymmetric vesicles", Proceedings of the National Academy of Sciences USA, 100: 10718-10721 (2003a) dar, welches auf dem von Träuble und Grell konzipierten Verfahren beruht, aber dieses um eine organische Zwischenphase erweitert, welche während des Zentrifugationsschrittes zwischen der Emulsion, welche die bereits mit einer monomolekularen Schicht aus zumindest einer amphiphilen Verbindung versehenen Pre-Liposomen enthält, und der wässrigen Phase, in welche die Pre-Liposomen unter Anlagerung der zweiten monomolekularen Schicht aus zumindest einer amphiphilen Verbindung überführt werden, vorhanden ist. Diese Zwischenphase ist zwar mit der organischen Wasser-in-Öl-Emulsion mit den Pre-Liposomen mischbar, aber aufgrund ihrer Dichtedifferenz im Zentrifugalfeld separierbar und enthält zumindest eine amphiphile Verbindung der äußeren Schicht der Bischicht, welche folglich von der amphiphilen Verbindung der inneren Monoschicht mehr oder minder getrennt ist. Durch die Zwischenphase entsteht auf diese Weise eine hinreichende Diffusionsgrenze für die amphiphilen Verbindungen an den Grenzschichten, womit die Bildung asymmetrischer Bischichten möglich ist. Allerdings besteht auch hier das Problem einer Ausbildung von Organogelen, insbesondere an der Phasengrenze zwischen der wässrigen Phase und der Zwischenphase. Überdies besteht insbesondere aufgrund dessen, dass die mit der (inneren) Monoschicht aus zumindest einer amphiphilen Verbindung versehenen Pre-Liposomen die Zwischenphase passieren müssen, bevor sich hieran die (äußere) Monoschicht der weiteren amphiphilen Verbindung anlagern kann, die Gefahr einer Kontamination der Liposomen mit der organischen Zwischenphase, welche nachträglich praktisch nicht beseitigt werden kann. Schließlich eignet sich das Verfahren gemäß Pautot et al. nicht für die Bildung von asymmetrischen Bischichten, bei welchen an einer Seite an die hydrophilen Kopfgruppen weitere hydrophile Konjugate, wie z.B. Polyethylenglykol (PEG) gebunden sind, weil gemäß dem Verfahren die zum Einbau in die äußere Monoschicht dienenden amphiphilen Verbindungen zuerst in der organischen Phase gelöst werden müssen, was die Löslichkeit von Konjugaten mit großen hydrophilen Molekülanteilen einschränkt.

Alle genannten Verfahren sind neben den beschriebenen ungelösten Problemen in der vorgestellten Form ferner noch nicht geeignet, um in einem kontinuierlichen Prozess angewandt zu werden, um sie dadurch im industriellen Maßstab in wirtschaftlicher Weise großtechnisch einsetzen zu können. Im Prinzip beruhen die Verfahren alle darauf, eine hydrophobe Phase gemeinsam mit einer wässrigen Phase zu zentrifugieren. Zum Abschluss des Herstellungsprozesses der Liposomen sind die beiden Phasen so zu separieren, dass die wässrige Phase als Produkt genutzt und die hydrophobe Phase als Hilfsstoff für die Durchführung des Prozesses wiederverwendet werden kann.

Der Erfindung liegt daher die Aufgabe zugrunde, unter zumindest weitestgehender Vermeidung der vorgenannten Nachteile ein einfaches und kostengünstiges Verfahren zur Verkapselung von Wirkstoffen in Liposomen vorzuschlagen, welches die Erzeugung von asymmetrischen Liposomen mit mehr oder minder frei einstellbaren, unterschiedlichen Zusammensetzungen der die Bischicht ihrer Membran bildenden Monoschichten aus amphiphilen Verbindungen ermöglicht, wobei die Ausbildung von Organogelen aus den die Bischicht bildenden amphiphilen Verbindungen minimiert werden und vorteilhafterweise auch eine kontinuierliche Herstellung von Liposomen ermöglicht sein sollte.

Erfindungsgemäß wird diese Aufgabe mit einem Verfahren zur Verkapselung von Wirkstoffen in Liposomen der eingangs genannten Art gelöst, welches die folgenden Schritte umfasst:
(a) Bereitstellen einer, insbesondere hydrophilen, Wirkstofflösung des zu verkapselnden Wirkstoffes, indem der Wirkstoff in wenigstens einem, insbesondere hydrophilen, Lösungsmittel gelöst wird;
(b) Bereitstellen einer ersten Emulsion durch Emulgieren der Wirkstofflösung gemäß Schritt (a) in wenigstens einer mit dem wenigstens einen Lösungsmittel der Wirkstofflösung nicht oder schlecht mischbaren, insbesondere hydrophoben, ersten Flüssigkeit in Gegenwart der wenigstens einen ersten amphiphilen Verbindung, um eine zumindest monomolekulare, innere Schicht der wenigstens einen ersten amphiphilen Verbindung an die in der ersten Flüssigkeit emulgierten Tropfen der Wirkstofflösung anzulagern;
(c) Bereitstellen einer Mischung aus einer mit der ersten Flüssigkeit der ersten Emulsion gemäß Schritt (b) nicht oder schlecht mischbaren, insbesondere hydrophilen, flüssigen Phase mit der wenigstens einen zweiten amphiphilen Verbindung;
(d) Inkontaktbringen der ersten Emulsion aus der ersten Flüssigkeit mit den hierin emulgierten Tropfen der Wirkstofflösung mit der hieran angelagerten, zumindest monomolekularen, inneren Schicht der wenigstens einen ersten amphiphilen Verbindung gemäß Schritt (b) mit der Mischung aus der flüssigen Phase mit der wenigstens einen zweiten amphiphilen Verbindung gemäß Schritt (c) unter Ausbildung einer Phasengrenze zwischen der ersten Emulsion gemäß Schritt (b) und der Mischung gemäß Schritt (c), wobei die wenigstens eine zweite amphiphile Verbindung an der Phasengrenze angereichert wird; und
(e) Zentrifugieren der über die Phasengrenze miteinander in Kontakt stehenden ersten Emulsion gemäß Schritt (b) und der Mischung gemäß Schritt (c), um die Tropfen der in der ersten Emulsion enthaltenen Wirkstofflösung mit der hieran angelagerten, zumindest monomolekularen, inneren Schicht der wenigstens einen ersten amphiphilen Verbindung aus der ersten Flüssigkeit der ersten Emulsion gemäß Schritt (b) in die flüssige Phase der Mischung gemäß Schritt (c) zu überführen, wobei beim Passieren der Phasengrenze die dort angereicherte, wenigstens eine zweite amphiphile Verbindung unter Bildung einer zumindest monomolekularen, äußeren Schicht derselben an die zumindest monomolekulare innere Schicht der wenigstens einen ersten amphiphilen Verbindung der Tropfen der Wirkstofflösung angelagert wird, um die Bischicht aus den beiden zumindest monomolekularen Schichten der wenigstens einen ersten amphiphilen Verbindung und der wenigstens einen zweiten amphiphilen Verbindung zu erzeugen.

Das erfindungsgemäße Verfahren zur Herstellung von Liposomen eignet sich in gleicher Weise sowohl zur Erzeugung von symmetrischen Liposomen (die erste amphiphile Verbindung bzw. die ersten amphiphilen Verbindungen ist/sind mit der zweiten amphiphilen Verbindung bzw. mit den zweiten amphiphilen Verbindungen identisch) als auch insbesondere zur Erzeugung von asymmetrischen Liposomen (die erste amphiphile Verbindung bzw. die ersten amphiphilen Verbindungen ist/sind von der zweiten amphiphilen Verbindung bzw. von den zweiten amphiphilen Verbindungen verschieden), wobei die erste(n) und zweite(n) amphiphilen Verbindungen praktisch frei wählbar sind, um jeweils eine Monoschicht aus individuell einstellbaren ersten bzw. zweiten amphiphilen Verbindungen zu erzeugen.

Entsprechend dem eingangs zitierten Stand der Technik wird bzw. werden also zunächst in als solcher bekannten Weise in einem Schritt (a) ein oder mehrere zu verkapselnde(r) Wirkstoff(e) in einem Lösungsmittel oder in einem Lösungsmittelgemisch gelöst, um eine Wirkstofflösung des wenigstens einen zu verkapselnden Wirkstoffes zu erzeugen. Bei dem wenigstens einen Lösungsmittel kann es sich z.B., wenngleich nicht notwendigerweise, insbesondere um ein hydrophiles Lösungsmittel handeln, wobei sich in der Praxis vornehmlich physiologisch unbedenkliche Lösungsmittel auf wässriger Basis einschließlich Wasser bzw. isotonischen Lösungen sowie beispielsweise auch alkoholische Lösungsmittel, insbesondere auf Basis von Ethanol, anbieten können.

In einem Schritt (b) wird - insoweit wiederum analog zu dem eingangs geschilderten Stand der Technik - diese Wirkstofflösung sodann unter Erhalt einer ersten Emulsion in einer mit dem wenigstens einen Lösungsmittel der Wirkstofflösung nicht oder schlecht mischbaren ersten Flüssigkeit oder mit einer Mischung aus solchen ersten Flüssigkeiten in Gegenwart einer oder mehrerer ersten amphiphilen Verbindung(en) emulgiert, so dass eine zumindest monomolekulare innere Schicht der wenigstens einen ersten amphiphilen Verbindung an die in der ersten Flüssigkeit emulgierten Tropfen der Wirkstofflösung angelagert wird. Auf diese Weise werden wiederum zunächst Pre-Liposomen im Sinne der vorliegenden Offenbarung erzeugt, deren monomolekulare Schicht der wenigstens einen ersten amphiphilen Verbindung die innere Schicht der Bischicht der in den nachfolgenden Schritten zu erzeugenden Liposomen bildet. Aufgrund dessen, dass die erste Emulsion jedoch in Abweichung zum Stand der Technik nur die wenigstens eine erste amphiphile Verbindung der inneren Monoschicht der Bischicht enthält, wird die Bildung von Pre-Liposomen, welche (auch) die wenigstens eine für die äußere Monoschicht der Bischicht vorgesehene, zweite amphiphile Verbindung enthalten, zuverlässig vermieden, so dass bereits insoweit eine größtmögliche Asymmetrie der Bischicht erzielt werden kann. Die mittlere Tropfengröße und -verteilung dieser ersten Emulsion und folglich der mittlere Durchmesser der Pre-Liposomen bzw. der hieraus zu erzeugenden Liposomen kann dabei in üblicher Weise z.B. durch entsprechenden Eintrag mechanischer Kräfte, wie z.B. Scherkräfte, bei der Erzeugung der ersten Emulsion eingestellt und in breiten Intervallen variiert werden. Während eines Zeitraumes von wenigen Minuten bis zu einigen Stunden vermag sich die wenigstens eine, zur Pre-Liposomen- bzw. Membranbildung befähigte erste amphiphile Verbindung dann als (innere) Monoschicht diffusiv an der Phasengrenze zwischen den Wirkstofflösungstropfen und der ersten Flüssigkeit der ersten Emulsion auszubilden. Sofern es sich bei der Wirkstofflösung um eine üblicherweise hydrophile, z.B. wässrige und/oder alkoholische, Lösung handelt, handelt es sich bei der bzw. den ersten Flüssigkeit(en) um hiermit nicht oder nur schlecht mischbare hydrophobe Flüssigkeiten. Bei der ersten amphiphilen Substanz kann es sich insbesondere um Lipide, wie z.B. um Phospholipide und dergleichen, oder auch um beliebige andere, zur Erzeugung von Liposomen bekannte amphiphile Verbindungen bzw. um Gemische solcher Verbindungen einschließlich jenen der eingangs genannten Art handeln.

Neben der Bereitstellung dieser ersten Emulsion mit den Pre-Liposomen aus in der inneren Schicht der wenigstens einen ersten amphiphilen Verbindung eingeschlossenen Tropfen der Wirkstofflösung wird nun in einem Schritt (c) ferner eine mit der ersten Flüssigkeit dieser ersten Emulsion, d.h. mit deren kontinuierlicher Phase, nicht oder schlecht mischbare Mischung bereitgestellt, wobei diese Mischung aus einer mit der ersten Flüssigkeit der ersten Emulsion gemäß dem obigen Schritt (b) nicht oder schlecht mischbaren flüssigen Phase mit der wenigstens einen zweiten amphiphilen Verbindung gebildet ist. In Abweichung zum Stand der Technik wird folglich nicht lediglich eine "reine" flüssige Phase, sondern eine Mischung aus einer solchen flüssigen Phase mit der wenigstens einen zweiten amphiphilen Verbindung bereitgestellt, welche im Rahmen der nachfolgenden Verfahrensschritte die zumindest monomolekulare äußere Schicht der Bischicht der zu erzeugenden Liposomen bildet, wobei die wenigstens eine zweite amphiphile Verbindung folglich räumlich getrennt von der wenigstens einen ersten amphiphilen Verbindung gehalten wird, welche in der ersten Emulsion vorliegt und dort die zumindest monomolekulare innere Schicht der dort emulgierten Pre-Liposomen bildet. Aufgrund dessen, dass diese Mischung nur die wenigstens eine zweite amphiphile Verbindung der äußeren Monoschicht der Bischicht enthält, wird die Bildung von Pre-Liposomen, welche (auch) die wenigstens eine für die innere Monoschicht der Bischicht vorgesehene, erste amphiphile Verbindung enthalten, vermieden, so dass auch insoweit eine größtmögliche Asymmetrie der Bischicht erzielt werden kann. Bei der flüssigen Phase dieser Mischung handelt es sich insbesondere um eine hydrophile Phase, sofern die wenigstens eine erste Flüssigkeit der die Pre-Liposomen enthaltenden ersten Emulsion, also deren kontinuierliche Phase, hydrophob bzw. die Wirkstofflösung hydrophil ist.

Sobald die (innere) monomolekulare Schicht der wenigstens einen ersten amphiphilen Verbindung bei der gemäß dem obigen Schritt (b) erzeugten ersten Emulsion eine Mindestdichte erreicht hat, mit welcher sie später eine Bischicht zu bilden vermag, wird in einem darauffolgenden Schritt (d) sodann die erste Emulsion mit den hierin emulgierten Tropfen der Wirkstofflösung mit der hieran angelagerten, zumindest monomolekularen, inneren Schicht der wenigstens einen ersten amphiphilen Verbindung, also mit den im obigen Schritt (b) erzeugten Pre-Liposomen, mit der Mischung aus der flüssigen Phase mit der wenigstens einen zweiten amphiphilen Verbindung gemäß dem obigen Schritt (c) unter Ausbildung einer Phasengrenze zwischen dieser ersten Emulsion und dieser Mischung in Kontakt gebracht, wobei zumindest die wenigstens eine zweite amphiphile Verbindung an dieser Phasengrenze angereichert wird. Eine etwaige Anreicherung auch eines Überschusses der wenigstens einen ersten amphiphilen Verbindung aus der ersten Emulsion an der Phasengrenze kann dabei auf einfache Weise zumindest weitestgehend verhindert werden, indem die wenigstens eine zweite amphiphile Verbindung der Mischung im Überschuss zugesetzt wird und/oder die wenigstens eine erste amphiphile Verbindung der ersten Emulsion mit einem Anteil zugesetzt wird, welcher etwa dem Anteil entspricht, welcher sich an die dort emulgierten Tropfen der Wirkstofflösung als innere Monoschicht anzulagern vermag.

In einem abschließenden Schritt (e) erfolgt schließlich das Zentrifugieren der über die Phasengrenze miteinander in Kontakt stehenden ersten Emulsion gemäß dem obigen Schritt (b) und der Mischung gemäß dem obigen Schritt (c), um die Tropfen der in der ersten Emulsion enthaltenen Wirkstofflösung mit der hieran angelagerten, zumindest monomolekularen, inneren Schicht der wenigstens einen ersten amphiphilen Verbindung, also den in der obigen Weise vorgefertigten Pre-Liposomen im Sinne der vorliegenden Offenbarung, aus der ersten Flüssigkeit der ersten Emulsion in die flüssige Phase der Mischung zu überführen, wobei beim Passieren der Phasengrenze die dort angereicherte, wenigstens eine zweite amphiphile Verbindung unter Bildung einer zumindest monomolekularen, äußeren Schicht derselben an die zumindest monomolekulare, innere Schicht der wenigstens einen ersten amphiphilen Verbindung der Tropfen der Wirkstofflösung angelagert wird, um die Bischicht aus den beiden zumindest monomolekularen Schichten der wenigstens einen ersten und zweiten amphiphilen Verbindung zu erzeugen. Die Pre-Liposomen aus den Tropfen der Wirkstofflösung mit der hieran angelagerten monomolekularen (inneren) Schicht der wenigstens einen ersten amphiphilen Verbindung werden folglich im Zentrifugalkraftfeld aufgrund ihrer Dichtedifferenz aus der ersten, insbesondere hydrophoben, Flüssigkeit der ersten Emulsion, also aus deren kontinuierlicher Phase, zu der über die Phasengrenze an diese angrenzende, insbesondere hydrophile, flüssige Phase der Mischung bewegt. An dieser Phasengrenze wird dann kontinuierlich die wenigstens eine zweite amphiphile Verbindung als monomolekulare (äußere) Schicht an die Pre-Liposomen angelagert, um die fertigen, mit einer Bischicht versehenen Liposomen zu bilden. Dabei nähern sich die beiden zumindest monomolekularen (inneren und äußeren) Schichten der wenigstens einen ersten und der wenigstens einen zweiten amphiphilen Verbindung zunächst so dicht aneinander an, dass sie aufgrund von, insbesondere hydrophoben, Wechselwirkungen zwischen den - selben (sofern symmetrische Liposomen erzeugt werden sollen) oder insbesondere unterschiedlichen (sofern asymmetrische Liposomen erzeugt werden sollten) - ersten und zweiten amphiphilen Verbindungen der Monoschichten die fertige Bischicht mit praktisch frei einstellbarer Komposition der inneren und äußeren Seite bilden. Sodann werden die eingekapselten Tropfen der Wirkstofflösung aus der ersten Emulsion aufgrund der Dichtedifferenz so stark gegen die neugebildete Bischicht gedrückt, dass sich letztere deformiert und schließlich den gesamten Wirkstofflösungstropfen umgibt, welcher sich schließlich von der Bischicht abschnürt und von der Phasengrenze abreißt, so dass durch die Umhüllung mit einer Bischicht der Wirkstofflösungstropfen in ein Liposom transformiert wird. Wird als wenigstens eine zweite amphiphile Verbindung dabei eine von der wenigstens einen ersten amphiphilen Verbindung verschiedene Verbindung gewählt, so lassen sich in einfacher Weise asymmetrische Liposomen mit einem unterschiedlichen Aufbau der inneren und äußeren, zumindest monomolekularen Schicht ihrer Bischicht erzeugen.

Der Vollständigkeit halber sei an dieser Stelle darauf hingewiesen, dass mit "nicht oder schlecht mischbar" in Bezug auf die (hydrophile) Wirkstofflösung mit der (hydrophoben) ersten Flüssigkeit der ersten Emulsion bzw. in Bezug auf die (hydrophobe) erste Flüssigkeit der ersten Emulsion mit der Mischung aus der (hydrophilen) flüssigen Phase und der wenigstens einen zweiten amphiphilen Verbindung eine hinreichend schlechte Mischbarkeit angesprochen ist, welche bei Vereinigung der vorgenannten Komponenten zur Bildung einer Phasengrenze führt.

Gemäß einer vorteilhaften Ausgestaltung des erfindungsgemäßen Verfahrens kann vorgesehen sein, dass
- als Mischung aus der, insbesondere hydrophilen, flüssigen Phase mit der wenigstens einen zweiten amphiphilen Verbindung gemäß Schritt (c) eine zweite Emulsion aus der flüssigen Phase und wenigstens einer hiermit nicht oder schlecht mischbaren, insbesondere hydrophoben, zweiten Flüssigkeit mit der wenigstens einen zweiten amphiphilen Verbindung eingesetzt wird, indem die zweite Flüssigkeit in der flüssigen Phase in Gegenwart der wenigstens einen zweiten amphiphilen Verbindung emulgiert wird, um eine zumindest monomolekulare Schicht der wenigstens einen zweiten amphiphilen Verbindung an die in der flüssigen Phase emulgierten Tropfen der zweiten Flüssigkeit anzulagern und die zweite amphiphile Verbindung auf diese Weise an diesen Tropfen zu immobilisieren; und
- beim Zentrifugieren der über die Phasengrenze miteinander in Kontakt stehenden ersten Emulsion gemäß Schritt (b) und der in Form der zweiten Emulsion vorliegenden Mischung gemäß Schritt (c) die Tropfen der zweiten Flüssigkeit mit der hieran angelagerten, zumindest monomolekularen Schicht der wenigstens einen zweiten amphiphilen Verbindung aus der flüssigen Phase der zweiten Emulsion fortwährend an die Phasengrenze zwischen der ersten Emulsion und der zweiten Emulsion überführt werden, um die wenigstens eine zweite amphiphile Verbindung fortwährend an der Phasengrenze anzureichern.

Anstelle einer bloßen Mischung oder Suspension der wenigstens einen zweiten amphiphilen Verbindung mit der flüssigen Phase gemäß Schritt (c) wird folglich eine zweite Emulsion eingesetzt, indem die, insbesondere hydrophile, flüssige Phase dieser Mischung mit der wenigstens einen hiermit nicht oder schlecht mischbaren, insbesondere hydrophoben, zweiten Flüssigkeit emulgiert wird. Auf diese Weise lassen sich in der zweiten Emulsion - in entsprechender Weise wie in der ersten Emulsion - Emulsionstropfen erzeugen, welche aus den in der flüssigen Phase emulgierten Tropfen der zweiten Flüssigkeit und einer hieran angelagerten, zumindest monomolekularen Schicht der wenigstens einen zweiten amphiphilen Verbindung gebildet sind. Die wenigstens eine zweite amphiphile Verbindung kann auf diese Weise immobilisiert werden und liegt folglich nicht mehr oder minder frei in einer bloßen Mischung bzw. Suspension vor. Wird diese zweite Emulsion gemäß dem Schritt (d) mit der ersten Emulsion unter Ausbildung einer Phasengrenze zwischen deren kontinuierlichen Phasen miteinander in Kontakt gebracht, so werden bei der darauffolgenden Zentrifugation der ersten und der zweiten Emulsion entsprechend dem Schritt (e) die Tropfen der zweiten Flüssigkeit mit der hieran angelagerten, zumindest monomolekularen Schicht der wenigstens einen zweiten amphiphilen Verbindung aus der flüssigen Phase der zweiten Emulsion fortwährend an die Phasengrenze zwischen der ersten Emulsion und der zweiten Emulsion überführt, so dass eine übermäßige Anreicherung der wenigstens einen zweiten amphiphilen Verbindung, geschweige denn die Bildung eines Organogels aus derselben, an der Phasengrenze zuverlässig verhindert werden kann, weil die an den emulgierten Tropfen der zweiten Flüssigkeit immobilisierte, wenigstens eine zweite amphiphile Verbindung auf diese Weise fortwährend der Phasengrenze zugeführt und an dieser Phasengrenze kontinuierlich angereichert wird, um sich beim Durchtritt der Tropfen aus der Wirkstofflösung mit der hieran angelagerten, zumindest monomolekularen (inneren) Schicht aus der wenigstens einen ersten amphiphilen Verbindung durch die Phasengrenze hindurch als äußere Schicht an die innere Schicht anzulagern und die Bischicht zu bilden. Darüber hinaus lassen sich auf diese Weise insbesondere die erste und die zweite amphiphile Verbindung in idealer Weise voneinander getrennt halten (beide amphiphile Verbindung sind jeweils an Emulsionstropfen der nur über die Phasengrenze miteinander in Kontakt stehenden ersten und zweiten Emulsion angelagert und folglich immobilisiert), so dass höchst asymmetrische Liposomen erzeugt werden können, sofern die zweite(n) amphiphile(n) Verbindung(en) von der/den ersten amphiphilen Verbindung(en) verschieden gewählt ist/sind.

Die in der zweiten Emulsion emulgierten Tropfen aus der, insbesondere hydrophoben, zweiten Flüssigkeit mit der hieran angelagerten, zumindest monomolekularen Schicht aus der wenigstens einen zweiten amphiphilen Verbindung werden im Folgenden der Einfachheit halber auch als "Amphiphil-Träger" bezeichnet.

Gemäß einer vorteilhaften Ausgestaltung des erfindungsgemäßen Verfahrens kann ferner vorgesehen sein, dass die erste Flüssigkeit der ersten Emulsion gemäß dem obigen Schritt (b) derart gewählt wird, dass die Löslichkeit der wenigstens einen ersten amphiphilen Verbindung in der ersten Flüssigkeit höchstens etwa 1 × 10⁻⁵ mol/l, vorzugsweise höchstens etwa 0,5 × 10⁻⁵ mol/l, höchst vorzugsweise höchstens etwa 1 × 10⁻⁶ mol/l, insbesondere höchstens etwa 1 × 10⁻⁷ mol/l, beträgt. In diesem Zusammenhang wurde überraschenderweise gefunden, dass eine Anreicherung der wenigstens einen ersten amphiphilen Verbindung an der Phasengrenze zwischen der ersten Emulsion und der hiermit in Kontakt stehenden flüssigen Phase der Mischung mit der wenigstens einen zweiten amphiphilen Verbindung bzw. der zweiten Emulsion, geschweige denn die Bildung eines Organogels, wie es sich bei dem eingangs geschilderten Stand der Technik als problematisch erweisen kann, in wirksamer Weise dadurch minimiert werden kann, dass die erste Flüssigkeit der ersten Emulsion gemäß dem obigen Schritt (b) derart gewählt wird, dass die Löslichkeit der wenigstens einen ersten amphiphilen Verbindung in der ersten Flüssigkeit sehr niedrig ist. Es kann sich auf diese Weise keine "Sperrschicht" aus der wenigstens einen ersten amphiphilen Verbindung an der Phasengrenze ausbilden, welche die Phasengrenze blockieren und damit die Bildung von Liposomen aus den Wirkstofflösungstropfen beim Durchtritt durch die Phasengrenze verhindern könnte, so dass eine hohe Ausbeute an mit einer Bischicht aus der wenigstens einen ersten und zweiten amphiphilen Verbindung versehenen Liposomen erhalten und der Wirkungsgrad des Verfahren somit in erheblicher Weise verbessert wird, indem der Ausschuss an Wirkstofflösung, welcher aus wirtschaftlichen Gründen zurückgewonnen werden sollte, minimiert wird.

Um auch eine übermäßige Anreicherung der wenigstens einen zweiten amphiphilen Verbindung an der vorgenannten Phasengrenze und folglich die Bildung eines Organogels aus derselben zu verhindern, kann es sich darüber hinaus insbesondere anbieten, dass die erste Flüssigkeit der ersten Emulsion gemäß Schritt (b) derart gewählt wird, dass die Löslichkeit sowohl der wenigstens einen ersten amphiphilen Verbindung als auch der wenigstens einen zweiten amphiphilen Verbindung in der ersten Flüssigkeit höchstens etwa 1 × 10⁻⁵ mol/l, vorzugsweise höchstens etwa 0,5 × 10⁻⁵ mol/l, höchst vorzugsweise höchstens etwa 1 × 10⁻⁶ mol/l, insbesondere höchstens etwa 1 × 10⁻⁷ mol/l, beträgt.

Die mit der, insbesondere hydrophilen, flüssigen Phase der zweiten Emulsion nicht oder schlecht mischbare, insbesondere hydrophobe, zweite Flüssigkeit, welche den "Kern" der Amphiphil-Träger im Sinne der vorliegenden Offenbarung mit der hieran angelagerten, wenigstens einen zweiten amphiphilen Verbindung bildet, kann vorteilhafterweise entsprechend der, insbesondere hydrophoben, ersten Flüssigkeit der ersten Emulsion gemäß Schritt (b) gewählt werden, welche die kontinuierliche Phase der ersten Emulsion bildet. Entspricht die erste Flüssigkeit demnach der zweiten Flüssigkeit, so kann diese in problemloser Weise in stets gleichbleibender Zusammensetzung wiedergewonnen werden, nachdem während des Zentrifugierens die Amphiphil-Träger aus der zweiten Flüssigkeit und der hieran angelagerten, wenigstens einen zweiten amphiphilen Verbindung die Phasengrenze zwischen der ersten Emulsion und der zweiten Emulsion erreicht haben, die zweite amphiphile Verbindung an der Phasengrenze angereichert und als äußere Schicht an die Pre-Liposomen aus der Wirkstofflösung mit der hieran angelagerten, wenigstens einen ersten amphiphilen Verbindung angelagert worden ist und die Tropfen aus der zweiten Flüssigkeit der zweiten Emulsion über die Phasengrenze hinweg in - dieselbe - erste Flüssigkeit übergegangen sind.

Die in Form der zweiten Emulsion vorliegende Mischung mit den an den Amphiphil-Trägern immobilisierten, wenigstens einen zweiten amphiphilen Verbindung gemäß dem Schritt (c) kann vorzugsweise dadurch erzeugt werden, indem
- zunächst eine Mischung aus der flüssigen Phase gemäß Schritt (c) und der wenigstens einen zweiten amphiphilen Verbindung bereitgestellt wird; wonach
- die zweite Flüssigkeit in dieser Mischung unter Bildung der zweiten Emulsion emulgiert wird, indem die zweite Flüssigkeit in diese Mischung eindispergiert wird.
Auf diese Weise werden aus der in der Mischung der, insbesondere hydrophilen, flüssigen Phase bereits enthaltenen, wenigstens einen zweiten amphiphilen Verbindung in zuverlässiger und reproduzierbarer Weise Amphiphil-Träger im Sinne der vorliegenden Offenbarung gebildet, wenn die, insbesondere hydrophobe, zweite Flüssigkeit in diese Mischung eindispergiert wird und sich an deren Tropfen die zweiten amphiphile Verbindung anlagert.

In entsprechender Weise kann die erste Emulsion gemäß dem Schritt (b) in vorteilhafter Weise dadurch erzeugt wird, indem zunächst
- eine Mischung aus der Wirkstofflösung des zu verkapselnden Wirkstoffes gemäß Schritt (a) und der wenigstens einen ersten amphiphilen Verbindung bereitgestellt wird; wonach
- diese Mischung unter Bildung der ersten Emulsion gemäß Schritt (b) in der ersten Flüssigkeit emulgiert wird, indem die Mischung in die erste Flüssigkeit eindispergiert wird.
Für die Gleichmäßigkeit der Größe der Liposomen kann die Größenverteilung der ersten Emulsion dabei durch eine entsprechende mechanische Zerkleinerung eingestellt werden. Für die Minimierung der Größe der Wirkstofflösungstropfen und die Optimierung ihrer Monodispersität und folglich der Liposomen kann es von Vorteil sein, wenn mechanische Zerkleinerungsverfahren entweder kontinuierlich oder zyklisch angewandt werden, um den Energieeintrag durch eine Temperierung auszugleichen. Entsprechendes gilt für die etwaige Erzeugung der zweiten Emulsion (siehe oben).

Wie bereits erwähnt, kann es sich bei der Auswahl der ersten, insbesondere hydrophoben, Flüssigkeit der ersten Emulsion gemäß Schritt (b) als vorteilhaft erweisen, wenn die Löslichkeit der wenigstens einen ersten amphiphilen Verbindung und vorzugsweise auch der wenigstens einen zweiten amphiphilen Verbindung höchstens etwa 1 × 10⁻⁵ mol/l beträgt, so dass die Bildung von Organogelstrukturen an der Phasengrenze zwischen der ersten Flüssigkeit der ersten Emulsion und der Mischung aus der, insbesondere hydrophilen, flüssigen Phase und der wenigstens einen zweiten amphiphilen Verbindung bzw. der zweiten Emulsion zuverlässig vermieden wird. Weist die Wirkstofflösung dabei einen hydrophilen Charakter auf und liegt beispielsweise in wässriger und/oder alkoholischer Form vor, so kann als erste Flüssigkeit der ersten Emulsion gemäß Schritt (b) zweckmäßigerweise eine hydrophobe Flüssigkeit eingesetzt, welche insbesondere aus der Gruppe der
- flüssigen halogenierten Kohlenwasserstoffe einschließlich der Fluorcarbone,
- Silikonöle und
- Siloxane
einschließlich Mischungen hiervon gewählt wird, in welcher insbesondere praktisch alle amphiphilen Lipide die erfindungsgemäß nur sehr geringe Löslichkeit aufweisen. Fluorierte oder perfluorierte Öle, d.h. Fluorcarbone, sind praktisch nicht mit Wasser oder anderen hydrophilen Flüssigkeiten mischbare Phasen, welche alle zur Implementierung des erfindungsgemäßen Verfahrens notwendigen Eigenschaften mitbringen, wie bezüglich einer unterschiedlichen, insbesondere erhöhten, Dichte gegenüber wässrigen und alkoholischen Medien, eines tiefe(re)n Gefrierpunktes (als wässrige und alkoholische Medien), einer relativ geringen Viskosität und eben insbesondere der Vermeidung von gelbildenden Interaktionen mit amphiphilen Verbindungen einschließlich Lipiden und einer hydrophilen, z.B. wässrigen und/oder alkoholischen, Phase. Entsprechendes gilt für Silikonöle, womit polymerisierte Siloxane mit organischen Seitenketten in flüssiger Form angesprochen sind, und für Siloxane in flüssiger Form, womit Verbindungen mit der allgemeinen Struktur 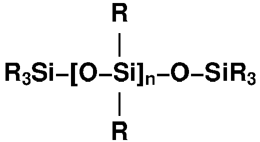 angesprochen sind, wobei jedes "R" ein Wasserstoffatom oder eine Alkylgruppe und "n" eine natürliche Zahl ist.

Nicht nur in diesem Fall wird als Lösungsmittel der Wirkstofflösung gemäß Schritt (a) folglich vorzugsweise ein hydrophiles Lösungsmittel, insbesondere auf wässriger Basis, einschließlich Wasser, wie beispielsweise isotonische Lösungen, und/oder auf alkoholischer Basis, insbesondere auf Basis von Ethanol oder Glycerin bzw. Glycerol, eingesetzt.

Gemäß einer Weiterbildung des erfindungsgemäßen Verfahrens kann vorgesehen sein, dass
- die erste Flüssigkeit der ersten Emulsion aus der ersten Flüssigkeit mit den hierin emulgierten Tropfen der Wirkstofflösung mit der hieran angelagerten, zumindest monomolekularen, inneren Schicht der wenigstens einen ersten amphiphilen Verbindung gemäß Schritt (b) derart gewählt wird, dass sie einen niedrigeren Schmelzpunkt aufweist als die Wirkstofflösung;
- die erste Emulsion gemäß Schritt (b) auf eine Temperatur zwischen dem Schmelzpunkt der ersten Flüssigkeit der ersten Emulsion und dem Schmelzpunkt der Wirkstofflösung abgekühlt wird, um die in der ersten Flüssigkeit emulgierten Tropfen der Wirkstofflösung mit der an diese angelagerten, zumindest monomolekularen, inneren Schicht der wenigstens einen ersten amphiphilen Verbindung gemäß Schritt (b) in den festen Zustand zu überführen; und sodann
- die erste Emulsion gemäß Schritt (b) im festen Zustand der Tropfen der Wirkstofflösung mit der mit der ersten Flüssigkeit der ersten Emulsion gemäß Schritt (b) nicht oder schlecht mischbaren, insbesondere hydrophoben, flüssigen Phase der Mischung bzw. der zweiten Emulsion mit der wenigstens einen zweiten amphiphilen Verbindung gemäß Schritt (c) unter Ausbildung der Phasengrenze gemäß Schritt (d) in Kontakt gebracht wird und die über die Phasengrenze miteinander in Kontakt stehenden erste Emulsion und die Mischung bzw. die zweite Emulsion gemäß Schritt (e) zentrifugiert werden.

Hierbei bietet es sich vorteilhafterweise an, wenn die, insbesondere hydrophile, Wirkstofflösung mit der an diese angelagerten, zumindest monomolekularen, inneren Schicht der wenigstens einen ersten amphiphilen Verbindung während des Zentrifugierens im festen Zustand gehalten wird, um sie aufgrund dadurch bedingten Dichteunterschiedes von der Phasengrenze fort in Richtung der Mischung bzw. der zweiten Emulsion zu bewegen.

Die erste Emulsion wird folglich vorzugsweise so stark abgekühlt, dass die, z.B. wässrigen und/oder alkoholischen, Wirkstofflösungstropfen einfrieren, die diese umgebende, insbesondere hydrophobe, erste Flüssigkeit, also die kontinuierliche Phase der ersten Emulsion, jedoch noch flüssig bleibt. Dies vermag dazu beizutragen, dass die Wirkstofflösungstropfen mit der hieran angelagerten (inneren), zumindest monomolekularen Schicht der wenigstens einen ersten amphiphilen Verbindung an der Phasengrenze nicht übermäßig oder gar vollständig deformiert werden, ohne die Phasengrenze zu penetrieren und damit ihrerseits eine Sperrschicht an der Phasengrenze zu bilden. Wird dabei insbesondere eine, insbesondere hydrophobe, erste Flüssigkeit ausgewählt, welche schwerer ist als die beispielsweise wässrigen und/oder alkoholischen Wirkstofflösungstropfen, so lassen sich die gefrorenen Wirkstofflösungstropfen infolge Zentrifugalkräften nicht nur einfacher bzw. schneller in der ersten Flüssigkeit der ersten Emulsion bewegen, sondern auch in der, insbesondere hydrophilen, flüssigen Phase der Mischung mit der wenigstens einen zweiten amphiphilen Verbindung bzw. in der zweiten Emulsion, nachdem ihr Durchtritt durch die Phasengrenze erfolgt und die Umhüllung mit einer Bischicht unter Bildung der fertigen Liposomen stattgefunden hat, weil die gefrorenen Wirkstofflösungstropfen eine geringere Dichte besitzen als die, insbesondere hydrophile, nicht gefrorene flüssige Phase der Mischung mit der wenigstens einen zweiten amphiphilen Verbindung bzw. der zweiten Emulsion, wobei diese flüssige Phase ebenso wie das Lösungsmittel der Wirkstofflösungstropfen insbesondere wässrig bzw. alkoholisch oder von Wasser gebildet sein kann. Zumindest die erste Emulsion sollte zu diesem Zweck vorzugsweise geringfügig unterhalb des Gefrierpunktes der Wirkstofflösungstropfen temperiert werden, damit diese in der sie umgebenden ersten Flüssigkeit der ersten Emulsion gefroren bleiben.

Wie bereits erwähnt, können die in ein Liposom eingeschlossenen Wirkstoffe durch die Bischicht aus amphiphilen Verbindungen, wie in Form einer Bischicht aus denselben oder insbesondere aus unterschiedlichen Lipiden, auf dem Weg zu ihrem Bestimmungsort im Organismus gegen die zerstörende Wirkung von Enzymen und vor vorzeitiger Ausscheidung aus dem Körper geschützt werden. In manchen pharmazeutischen Produkten müssen die Liposomen allerdings auch durch eine oberflächige Polymerschicht, welche typischerweise auf der Basis von Polyethylenglykol (PEG) gebildet ist, geschützt werden, um die Opsonierung und Phagozytose durch Immunzellen, z.B. in der Leber, zu vermeiden, bevor der Wirkstoff an seinem Bestimmungsort angekommen ist. Mit Hilfe von Fremdmolekülen, wie beispielsweise Antikörpern, welche an das Äußere der Liposomen angeheftet werden, kann überdies versucht werden, den Bestimmungsort des Wirkstoffes durch Bindung an einen spezifischen Rezeptor genau festzulegen ("Drug Targeting"). Dabei wird vermutet, dass Liposomen aufgrund ihrer zellmembranähnlichen chemischen Beschaffenheit relativ leicht mit der Zellmembran oder, nach einer Pinocytose oder Endocytose, mit der Endosomen- und Lysosomenmembran verschmelzen und sodann ihren Inhalt in das Zellinnere entlassen. Demgemäß kann gemäß einer vorteilhaften Weiterbildung des erfindungsgemäßen Verfahrens vorgesehen sein, dass die Bischicht aus den beiden zumindest monomolekularen Schichten der wenigstens einen ersten amphiphilen Verbindung und der wenigstens einen zweiten amphiphilen Verbindung, insbesondere ausschließlich die äußere, zumindest monomolekulare Schicht der wenigstens einen zweiten amphiphilen Verbindung, durch Reaktion mit hydrophilen Polymerkonjugaten modifiziert wird. Dies kann beispielsweise einerseits dadurch geschehen, dass das fertig erzeugte Liposom derart modifiziert wird, indem solche Polymerkonjugate z.B. mittels elektrostatischer Aufladung der Bischicht an deren zumindest monomolekulare äußere Schicht angelagert werden, wie es als solches aus dem Stand der Technik bekannt ist. Darüber hinaus eröffnet das erfindungsgemäße Verfahren insbesondere die Möglichkeit, dass für die zumindest monomolekulare, äußere Schicht der Bischicht wenigstens eine zweite amphiphile Verbindung, z.B. in Form von Lipiden, eingesetzt wird, an welche zuvor die Polymerkonjugate bereits angebunden worden sind.

Darüber hinaus kann das erfindungsgemäße Verfahren
- chargenweise in einer diskontinuierlichen Zentrifugiereinrichtung, z.B. batchweise unter Einsatz von herkömmlichen Zentrifugenröhrchen; oder
- semikontinuierlich in einer diskontinuierlichen Zentrifugiereinrichtung, z.B. gleichfalls batchweise, aber unter Zufuhr der ersten Emulsion und der hiermit nicht oder schlecht mischbaren flüssigen Phase oder der Mischung bzw. zweiten Emulsion über einen Zeitraum hinweg;
durchgeführt werden.

Im Falle einer chargenweisen Durchführung des Verfahrens in einer diskontinuierlichen Zentrifugiereinrichtung kann beispielsweise vorgesehen sein, dass einerseits die erste Emulsion gemäß Schritt (b), andererseits die Mischung bzw. die zweite Emulsion gemäß Schritt (c) der Zentrifugiereinrichtung aufgegeben werden, wonach diese zentrifugiert werden, wonach der Zentrifugiereinrichtung einerseits die flüssige Phase mit den mit der Bischicht aus den beiden zumindest monomolekularen Schichten der wenigstens einen ersten amphiphilen Verbindung und der wenigstens einen zweiten amphiphilen Verbindung versehenen Liposomen, andererseits die erste Flüssigkeit entnommen werden. Im Falle einer semikontinuierlichen Durchführung des Verfahrens in einer diskontinuierlichen Zentrifugiereinrichtung kann beispielsweise vorgesehen sein, dass einerseits die erste Emulsion gemäß Schritt (b), andererseits die Mischung bzw. die zweite Emulsion gemäß Schritt (c) der Zentrifugiereinrichtung über einen Zeitraum kontinuierlich aufgegeben und diese währenddessen zentrifugiert werden, wonach der Zentrifugiereinrichtung wiederum einerseits die flüssige Phase mit den mit der Bischicht aus den beiden zumindest monomolekularen Schichten der wenigstens einen ersten amphiphilen Verbindung und der wenigstens einen zweiten amphiphilen Verbindung versehenen Liposomen, andererseits die erste Flüssigkeit entnommen werden.

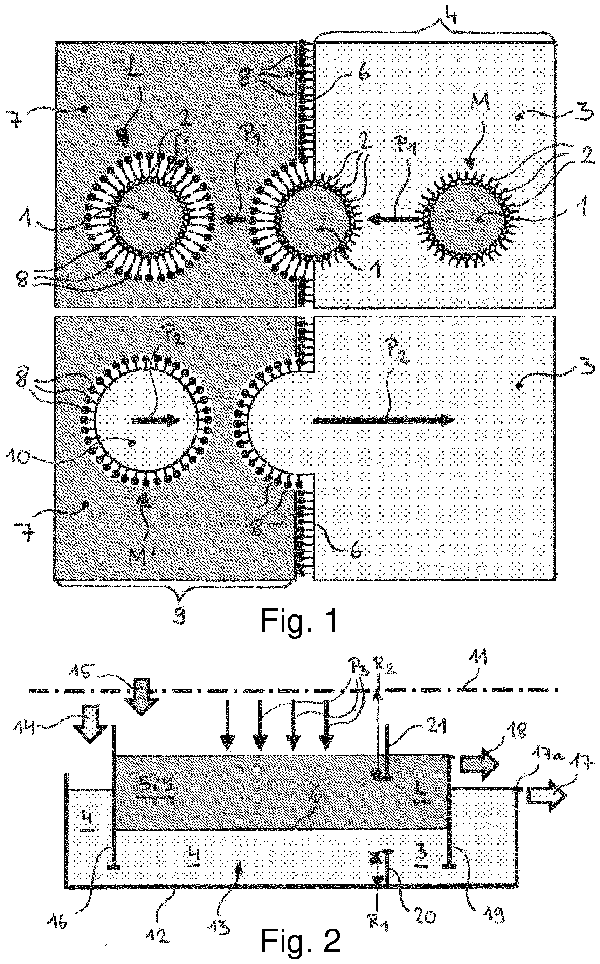

Wie auch weiter unten unter Bezugnahme auf die Fig. 2 noch im Einzelnen erläutert, kann stattdessen insbesondere auch vorgesehen sein, dass das erfindungsgemäße Verfahren kontinuierlich in einer im Durchfluss betriebenen, kontinuierlichen Zentrifugiereinrichtung durchgeführt wird, um eine wirtschaftliche und großtechnische Herstellung von symmetrischen oder insbesondere asymmetrischen Liposomen zu gewährleisten. Zu diesem Zweck kann beispielsweise einerseits die erste Emulsion gemäß Schritt (b), andererseits die Mischung bzw. die zweite Emulsion gemäß Schritt (c) der Zentrifugiereinrichtung kontinuierlich aufgegeben und zentrifugiert und der Zentrifugiereinrichtung einerseits die flüssige Phase mit den mit der Bischicht aus den beiden zumindest monomolekularen Schichten der wenigstens einen ersten amphiphilen Verbindung und der wenigstens einen zweiten amphiphilen Verbindung versehenen Liposomen, andererseits

die erste Flüssigkeit kontinuierlich entnommen werden. Dabei kann vorzugsweise vorgesehen sein, dass
- einerseits die erste Emulsion gemäß Schritt (b), andererseits die Mischung bzw. die zweite Emulsion gemäß Schritt (c) der kontinuierlichen Zentrifugiereinrichtung über separate Einlässe aufgegeben und in einem Einlassbereich der Zentrifugiereinrichtung, insbesondere mittels eines sich im Wesentlichen in Radialrichtung derselben erstreckenden Einlasswehres, voneinander getrennt gehalten werden, wonach sie infolge Passierens des Einlasswehres in eine gemeinsame Zentrifugierkammer überführt werden; und/oder
- einerseits die flüssige Phase mit den mit der Bischicht aus den beiden zumindest monomolekularen Schichten der wenigstens einen ersten amphiphilen Verbindung und der wenigstens einen zweiten amphiphilen Verbindung versehenen Liposomen, andererseits die erste Flüssigkeit aus einer gemeinsamen Zentrifugierkammer der Zentrifugiereinrichtung, insbesondere mittels eines sich im Wesentlichen in Radialrichtung derselben erstreckenden Auslasswehres, voneinander getrennt und der Zentrifugiereinrichtung über separate Auslässe entnommen werden.

Darüber hinaus kann es in diesem Zusammenhang von Vorteil sein, wenn
- die in der ersten Flüssigkeit emulgierten Tropfen der Wirkstofflösung mit der an diese angelagerten, zumindest monomolekularen, inneren Schicht der wenigstens einen ersten amphiphilen Verbindung der ersten Emulsion gemäß Schritt (b) in einer Zentrifugierkammer der kontinuierlichen Zentrifugiereinrichtung mittels eines in der Zentrifugierkammer, insbesondere in deren auslassseitigen Abschnitt, angeordneten und sich im Wesentlichen in Radialrichtung derselben erstreckenden ersten Rückhaltewehres akkumuliert werden; und/oder
- die in der flüssigen Phase der zweiten Emulsion emulgierten Tropfen der zweiten Flüssigkeit mit der hieran angelagerten, zumindest monomolekularen Schicht der wenigstens einen zweiten amphiphilen Verbindung in einer Zentrifugierkammer der kontinuierlichen Zentrifugiereinrichtung mittels eines in der Zentrifugierkammer, insbesondere in deren auslassseitigen Abschnitt, angeordneten und sich im Wesentlichen in Radialrichtung derselben erstreckenden zweiten Rückhaltewehres akkumuliert werden,
wie es beispielsweise weiter unten unter Bezugnahme auf die Fig. 2 näher erläutert ist.

Das erfindungsgemäße Verfahren - sei es zur Herstellung symmetrischer oder sei es insbesondere zur Herstellung asymmetrischer Liposomen - bietet eine neuartige Möglichkeit, technisch relativ einfach und kostengünstig Liposomen herzustellen, welche insbesondere für die therapeutische Nutzung, darüber hinaus aber für alle anderen bekannten Nutzungen von Liposomen, beispielsweise im Bereich der Nahrungsmittel oder auch der Kosmetik oder der Körperpflege, gleichermaßen geeignet sind. Die Anordnung von unterschiedlichen, zumindest monomolekularen Monoschichten an der Phasengrenze zwischen einer ersten Emulsion, wie einer "Wasser-in Öl-Emulsion", und einer zweiten Emulsion, wie einer "Öl-in Wasser- Emulsion", erlaubt es ferner, diese beiden Monoschichten durch hydrophobe Wechselwirkungen zwischen z.B. Fettsäureketten zu einer stabilen Bischicht mit unterschiedlichen Eigenschaften der späteren inneren bzw. äußeren Schicht eines Liposoms zusammenzufügen, wobei sich die Substanzkomposition der inneren und äußeren, zumindest monomolekularen Schicht der Bischicht frei einstellen lässt, was neue Möglichkeiten für die Funktionalität von Liposomen-Präparationen eröffnet. Zusätzlich ermöglicht der Herstellungsprozess eine hohe Einkapselungseffizienz, da durch die Erzeugung einer Emulsion in einer nicht mischbaren umgebenden Phase ein vollständiger Einschluss des Wirkstoffs in den Emulsionstropfen möglich ist. Bei der Umhüllung des Emulsionstropfens mit einer zweiten Monoschicht für die Einkapselung von Wirkstoffen in das dadurch neu erzeugte Liposom treten nur geringe Verluste des Wirkstoffes in den Umgebungsbereich des Liposoms nach dessen Herstellung auf, so dass eine hohe Nutzungsrate der üblicherweise teuren Wirkstoffe erzielt werden kann. Durch das Volumenverhältnis von hydrophiler und hydrophober Phase sowie durch das Mengenverhältnis der ersten und zweiten membranbildenden amphiphilen Verbindungen, z.B. in Form von Phospholipiden, können die Voraussetzungen für die Bildung von Liposomen in der gewünschten Größe und Konzentration geschaffen werden, wobei vorteilhafterweise ein entsprechender Überschuss an amphiphilen Verbindungen gewährleistet werden sollte, damit sich eine ausreichende Grenzflächendichte an amphiphilen Verbindungen an der Phasengrenze einstellt, was erfindungsgemäß jedoch nicht in der Ausbildung eines Organogels resultiert.

Weitere Merkmale und Vorteile der Erfindung ergeben sich aus der nachfolgenden Beschreibung eines Ausführungsbeispiels unter Bezugnahme auf die Zeichnungen. Dabei zeigen:
- Fig. 1: eine stark schematisiert dargestellte Ansicht zur Veranschaulichung der Erzeugung von asymmetrischen Liposomen mittels einer Ausführungsform eines erfindungsgemäßen Verfahrens zur Verkapselung von Wirkstoffen in Liposomen;
- Fig. 2: eine schematische Querschnittsansicht des halben Querschnitts einer Ausführungsform einer Zentrifugiereinrichtung zur kontinuierlichen Durchführung eines Verfahrens zur Verkapselung von Wirkstoffen in Liposomen;

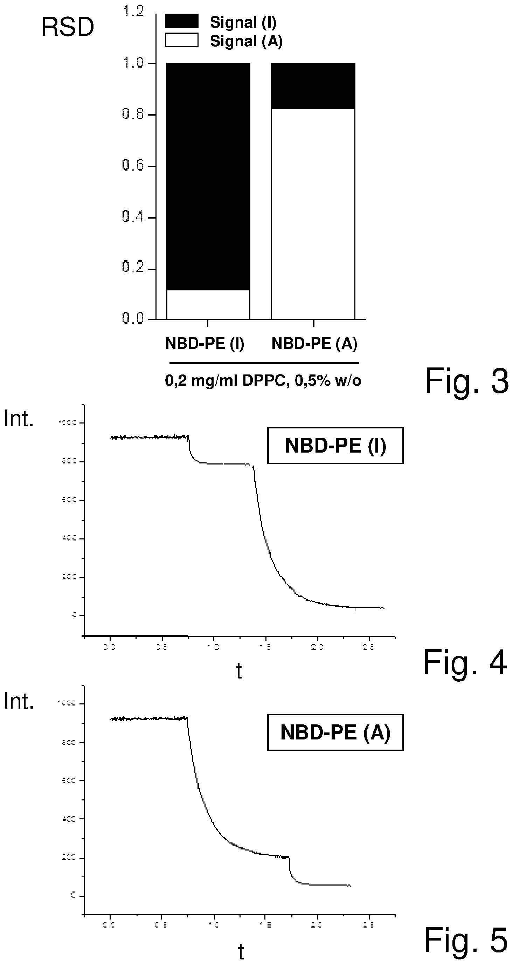

- Fig. 3: ein Schaubild zum Asymmetrienachweis von gemäß dem Ausführungsbeispiel 2 hergestellten asymmetrischen Liposomen, bei welchen einerseits nur die innere monomolekulare Schicht (I) aus der ersten amphiphilen Verbindung, andererseits nur die äußere monomolekulare Schicht (A) aus der zweiten amphiphilen Verbindung markiert worden ist, wobei die Fig. 3 die jeweilige relative Signalverteilung (relative signal distribution, RSD) der inneren Schicht (I) bzw. der äußeren Schicht (A) zeigt;
- Fig. 4: ein Schaubild zum Asymmetrienachweis von gemäß dem Ausführungsbeispiel 2 hergestellten asymmetrischen Liposomen, bei welchen nur die innere monomolekulare Schicht (I) aus der ersten amphiphilen Verbindung markiert worden ist, wobei die Fig. 4 die Abnahme der Signalintensität (Int.) über die Zeit (t) zeigt, wenn die Markierungssubstanz durch Zusetzen einer Abbausubstanz zerstört wird; und
- Fig. 5: ein Schaubild zum Asymmetrienachweis von gemäß dem Ausführungsbeispiel 2 hergestellten asymmetrischen Liposomen, bei welchen nur die äußere monomolekulare Schicht (A) aus der zweiten amphiphilen Verbindung markiert worden ist, wobei die Fig. 5 die Abnahme der Signalintensität (Int.) über die Zeit (t) zeigt, wenn die Markierungssubstanz durch Zusetzen einer Abbausubstanz zerstört wird.

In der Fig. 1 ist eine Situation während der Bildung von vornehmlich asymmetrischen Liposomen gemäß einer Ausführungsform eines erfindungsgemäßen Verfahrens zur Verkapselung von Wirkstoffen in Liposomen L anhand einer vergrößerten Detailansicht in stark schematisierter Weise wiedergegeben. Dabei ist in dem rechten oberen Ausschnitt der Fig. 1 ein Tropfen, z.B. mit einem Durchmesser zwischen etwa 0,1 µm und etwa 200 um, aus einer Wirkstofflösung 1 erkennbar, zu deren Erzeugung der Wirkstoff zuvor gemäß Schritt (a) in einem Lösungsmittel gelöst worden ist. Bei dem Lösungsmittel handelt es sich im vorliegenden Fall z.B. um ein hydrophiles Lösungsmittel auf wässriger und/oder alkoholischer Basis.

Wie des Weiteren in dem rechten oberen Ausschnitt der Fig. 1 erkennbar ist, hat sich an den Tropfen aus der Wirkstofflösung 1 unter Bildung eines Pre-Liposoms M eine monomolekulare (innere) Schicht aus einer ersten amphiphilen Verbindung 2, wie beispielsweise einem Lipid, angelagert, wobei sich die polaren Bereiche der ersten amphiphilen Verbindung 2 in Richtung der hydrophilen Wirkstofflösung 1 und die unpolaren Bereiche in Richtung einer den Tropfen der Wirkstofflösung 1 umgebenden, mit dessen Lösungsmittel nicht oder nur schlecht mischbaren - hier: hydrophoben - ersten Flüssigkeit 3 ausgerichtet haben. Zur Erzeugung einer derart gebildeten ersten Emulsion 4, deren disperse Phase von den Tropfen der hydrophilen Wirkstofflösung 1 und deren kontinuierliche Phase von der hydrophoben ersten Flüssigkeit 3 gebildet ist, ist zuvor gemäß Schritt (b) z.B. zunächst eine Mischung aus der Wirkstofflösung 1 und der ersten amphiphilen Verbindung 2 erzeugt worden, wonach diese Mischung unter Erhalt der ersten Emulsion 4 in die erste Flüssigkeit 3 eindispergiert worden ist. Die - hydrophobe - erste Flüssigkeit 3, bei welcher es sich im vorliegenden Fall beispielsweise um einen flüssigen halogenierten Kohlenwasserstoff in Form eines oder mehrerer Fluorcarbone handelt, ist dabei derart ausgewählt worden, dass die Löslichkeit der ersten amphiphilen Verbindung 2 kleiner als 10⁻⁵ mol/l beträgt (siehe hierzu weiter unten). Ferner ist die erste Flüssigkeit 3 vorzugsweise auch derart ausgewählt worden, dass sie einen geringeren Schmelzpunkt aufweist als die in der ersten Emulsion 4 emulgierte Wirkstofflösung 1 (siehe hierzu ebenfalls weiter unten). Die - hydrophobe - erste Flüssigkeit 3 besitzt ferner eine unterschiedliche Dichte als die Wirkstofflösung 1, wobei das im vorliegenden Fall eingesetzte Fluorcarbon eine höhere Dichte aufweist als die wässrige und/oder alkoholische Wirkstofflösung 1. Indes kann selbstverständlich grundsätzlich auch eine gegenüber der Wirkstofflösung 1 "leichtere" erste Flüssigkeit 3, also eine solche mit demgegenüber geringerer Dichte, eingesetzt werden.

Wie aus dem linken unteren Ausschnitt der Fig. 1 hervorgeht, ist darüber hinaus gemäß Schritt (c) eine Mischung 7 aus einer mit der ersten Flüssigkeit 3 der ersten Emulsion 4 nicht oder schlecht mischbaren - hier hydrophilen - flüssigen Phase mit einer zweiten amphiphilen Verbindung 8, beispielsweise wiederum in Form eines Lipides, bereitgestellt worden, wobei die flüssige Phase dieser Mischung 7 beispielsweise entsprechend dem Lösungsmittel der Wirkstofflösung 1 gewählt werden und folglich z.B. gleichfalls wässrig und/oder alkoholisch sein kann. Bei der Mischung 7 handelt es sich im vorliegenden Fall um eine zweite Emulsion 9 aus der (hydrophilen) flüssigen Phase der Mischung 7 und einer hiermit nicht oder schlecht mischbaren - hier hydrophoben - zweiten Flüssigkeit 10 und der zweiten amphiphilen Verbindung 8, welche dadurch erzeugt worden ist, dass die zweite (hydrophobe) Flüssigkeit 10 in der (hydrophilen) flüssigen Phase der Mischung 7 in Gegenwart der zweiten amphiphilen Verbindung 8 emulgiert worden ist, so dass die kontinuierliche Phase der zweiten Emulsion 9 von der (hydrophilen) flüssigen Phase und die disperse Phase der zweiten Emulsion 9 von der (hydrophoben) zweiten Flüssigkeit 10 gebildet ist. Letztere kann dabei insbesondere entsprechend der (hydrophoben) ersten Flüssigkeit 3 der ersten Emulsion 4 gewählt werden. Auf diese Weise wurde eine monomolekulare Schicht der zweiten amphiphilen Verbindung 8 an die in der (hydrophilen) flüssigen Phase der Mischung 7 emulgierten Tropfen der (hydrophoben) zweiten Flüssigkeit 10 angelagert und die zweite amphiphile Verbindung 8 folglich unter Bildung von Amphiphil-Trägern M' an diesen Tropfen immobilisiert.

Wie des Weiteren aus allen vier Ausschnitten der Fig. 1 ersichtlich, ist nun die erste Emulsion 4 aus der hydrophoben, ersten Flüssigkeit 3 mit den hierin emulgierten Pre-Liposomen M aus den Tropfen der Wirkstofflösung 1 mit der hieran angelagerten, monomolekularen (inneren) Schicht der ersten amphiphilen Verbindung 2 gemäß Schritt (b) mit der zweiten Emulsion 9 aus der hydrophilen flüssigen Phase mit den hierin emulgierten Amphiphil-Trägern M' aus den Tropfen der hydrophoben zweiten Flüssigkeit 10 mit der hieran angelagerten zweiten amphiphilen Verbindung 8 in Kontakt gebracht worden, woraufhin sich zwischen der ersten Flüssigkeit 3, also der kontinuierlichen Phase der ersten Emulsion 4, und der flüssigen Phase, also der kontinuierlichen Phase der zweiten Emulsion 9, aufgrund deren schlechter Mischbarkeit miteinander eine Phasengrenze 6 ausgebildet hat. Die zweite amphiphile Verbindung 8, welche der zweiten Emulsion 9 zu diesem Zweck vorzugsweise in einem gewissen Überschuss zugesetzt worden ist, hat sich dabei an dieser Phasengrenze 6 angereichert, wobei insbesondere aufgrund deren geringen Löslichkeit in der ersten Flüssigkeit 3 der ersten Emulsion 4 von weniger als etwa 10⁻⁵ mol/l gleichwohl kein Organogel infolge übermäßiger Akkumulierung der zweiten amphiphilen Verbindung 8 an der Phasengrenze 6 gebildet worden ist, sondern diese nur eine oder wenige Molekülschichten einnimmt. Die Anreicherung der ersten amphiphilen Verbindung 2, deren Löslichkeit in der ersten Flüssigkeit 3 der ersten Emulsion 4 gleichfalls weniger als etwa 10⁻⁵ mol/l beträgt, an der Phasengrenze 6 ist allenfalls sehr gering, da der Anteil der ersten amphiphilen Verbindung 2 einerseits derart eingestellt worden ist, dass sie möglichst vollständig als monomolekulare innere Schicht an die Tropfen der Wirkstofflösung 1 angelagert worden ist, andererseits verhindert die sehr geringe Löslichkeit der ersten amphiphilen Verbindung 2 in der ersten Flüssigkeit 3 der ersten Emulsion 4 eine übermäßige Anreicherung derselben an der Phasengrenze 6 oder gar die Bildung eines Organogels.

Schritt (e) des Verfahrens sieht schließlich eine Zentrifugation der über die Phasengrenze 6 miteinander in Kontakt stehenden ersten Emulsion 4 und der zweiten Emulsion 9 vor, um einerseits die Tropfen der in der ersten Emulsion 4 enthaltenen Wirkstofflösung 1 mit der hieran angelagerten monomolekularen inneren Schicht der ersten amphiphilen Verbindung 2 in Richtung der Pfeile P₁ aus der ersten Flüssigkeit 3 der ersten Emulsion 4 durch die Phasengrenze 6 mit den dort angereicherten Molekülen der zweiten amphiphilen Verbindung 8 hindurch in die flüssige, kontinuierliche Phase der zweiten Emulsion 9 zu überführen, wobei beim Passieren der Phasengrenze 6 die zweite amphiphile Verbindung 8 unter Bildung einer weiteren monomolekularen - äußeren - Schicht derselben an die monomolekulare innere Schicht der ersten amphiphilen Verbindung 2 der Tropfen der Wirkstofflösung 1 angelagert wird, um die Bischicht aus zwei monomolekularen Schichten, nämlich zum Einen der ersten amphiphilen Verbindung 2 (innere Schicht) und zum Anderen der zweiten amphiphilen Verbindung 8 (äußere Schicht) zu erzeugen, d.h. um aus den Pre-Liposomen M fertige Liposomen L zu bilden. Die äußere Schicht der Bischicht aus der zweiten amphiphilen Verbindung 8 weist dabei eine entgegengesetzte Orientierung wie die innere Schicht aus der ersten amphiphilen Verbindung 2 auf, d.h. die unpolaren Bereiche der zweiten amphiphilen Verbindung 8 der äußeren Schicht weisen in Richtung der polaren Bereiche der ersten amphiphilen Verbindung 2 der inneren Schicht, also in Richtung der nun in einem Liposom L verkapselten hydrophilen Wirkstofflösung 1, während die polaren Bereiche der zweiten amphiphilen Verbindung 8 der äußeren Schicht der Bischicht in Richtung der das Liposom L umgebenden - hydrophilen - flüssigen Phase der zweiten Emulsion 9 weisen (vgl. hierzu die beiden oberen Ausschnitte der Fig. 1).

Da die Pre-Liposomen M aus den Tropfen der (hydrophilen) Wirkstofflösung 1 mit der hieran angelagerten inneren Schicht der ersten amphiphilen Verbindung 2 eine geringere Dichte besitzen als die sie umgebende (hydrophobe) erste Flüssigkeit 3 der ersten Emulsion 4, erfahren sie im Zentrifugalfeld eine in Richtung der Pfeile P₁ wirkende Kraft, welche sie in zentripetaler Richtung beschleunigt und an die Phasengrenze 6 bringt, welche mit einer weitestgehend monomolekularen Schicht der zweiten amphiphilen Verbindung 8 belegt ist. Gegen diese, an der Phasengrenze 6 angereicherte Schicht der zweiten amphiphilen Verbindung 8 wird das Pre-Liposom M so gedrückt, dass sich die an der Phasengrenze 6 angereicherte monomolekulare Schicht der zweiten amphiphilen Verbindung 8 an die an dem Tropfen der Wirkstofflösung 1 angelagerte innere Schicht der ersten amphiphilen Verbindung 2 anlegt und dabei durch hydrophobe Wechselwirkung aus zwei monomolekularen Schichten die Bischicht des fertigen Liposoms L entsteht, welches nach weiterer Bewegung in Richtung der Pfeile P₁ im Zentrifugalfeld in der (hydrophilen) flüssigen Phase der zweiten Emulsion 9 dispergiert vorliegt. Bei einer vornehmlich kugelförmigen bzw. sphärischen Umhüllung entsteht folglich aus dem Tropfen der Wirkstofflösung 1 bzw. aus dem Pre-Liposom M ein Liposom L, welches in der seine Membran bildenden Bischicht zum Einen eine innere Schicht aus der ersten amphiphilen Verbindung 2 und zum Anderen eine äußere Schicht aus der zweiten amphiphilen Verbindung 8 aufweist. Folglich können einerseits symmetrische Liposomen L erzeugt werden, wenn die erste amphiphile Verbindung 2 entsprechend der zweiten amphiphilen Verbindung 8 gewählt wird; andererseits lassen sich insbesondere asymmetrische Liposomen L erzeugen, wenn die erste amphiphile Verbindung 2 von der zweiten amphiphilen Verbindung 8 verschieden gewählt wird. Indes sei an dieser Stelle darauf hingewiesen, dass anstelle einer (einzigen) ersten amphiphilen Verbindung 2 und/oder zweiten amphiphilen Verbindung 8 selbstverständlich auch ein Gemisch solcher Verbindungen, z.B. ein Gemisch aus mehreren Lipiden, eingesetzt werden kann, welche dann jeweils die innere bzw. die äußere Schicht der Bischicht des Liposoms L bilden (vgl. hierzu ebenfalls die beiden oberen Ausschnitte der Fig. 1).

Wie aus den beiden unteren Ausschnitten der Fig. 1 ferner ersichtlich, werden beim Zentrifugieren der über die Phasengrenze 6 miteinander in Kontakt stehenden ersten Emulsion 4 und der zweiten Emulsion 9 andererseits die Tropfen der zweiten Flüssigkeit 10 mit der hieran angelagerten monomolekularen Schicht der zweiten amphiphilen Verbindung 8, also die Amphiphil-Träger M', aus der flüssigen Phase der zweiten Emulsion 9 in Richtung der Pfeile P₂ fortwährend an die Phasengrenze 6 zwischen der ersten Emulsion 4 und der zweiten Emulsion 9 überführt, um die zweite amphiphile Verbindung 8 im Zentrifugalfeld fortwährend an der Phasengrenze 6 anzureichern, wo sie infolge der im vorherigen Absatz erläuterten Anlagerung als äußere Schicht an die Pre-Liposomen M unter Bildung der Liposomen L verbraucht wird.

Da die Amphiphil-Träger M' aus den Tropfen der (hydrophoben) zweiten Flüssigkeit 10 mit der hieran angelagerten zweiten amphiphilen Verbindung 8 eine höhere Dichte besitzen als die sie umgebende (hydrophile) flüssige Phase der zweiten Emulsion 9, erfahren sie im Zentrifugalfeld eine in Richtung der Pfeile P₂ wirkende Kraft, welche sie in zentrifugaler Richtung beschleunigt und an die Phasengrenze 6 bringt. Dabei streifen sie ihre monomolekulare Schicht aus der zweiten amphiphilen Verbindung 8 beim Übertritt in die erste (hydrophobe) Flüssigkeit 3, also in die kontinuierliche Phase der ersten Emulsion 4, an der Phasengrenze 6 ab. Auf diese Weise wird die an der Phasengrenze 6 abgeschiedenen Schicht aus der zweiten amphiphilen Verbindung 8 infolge des Phasenübertrittes der Tropfen aus der zweiten (hydrophoben) Flüssigkeit fortwährend erneuert bzw. wird die zweite amphiphile Verbindung 8 kontinuierlich an die Phasengrenze 6 "nachgeliefert", wohingegen sie durch die oben beschriebene Anlagerung als äußere Schicht unter Bildung der Liposomen L kontinuierlich verbraucht wird.

Nach mehr oder minder vollständiger Bildung der Liposomen L bzw. nach mehr oder minder vollständigem Verbrauch der in der zweiten Emulsion 9 ursprünglich bereitgestellten Amphiphil-Träger M' aus der zweiten Flüssigkeit 10 mit der hieran angelagerten zweiten amphiphilen Verbindung 8 kann schließlich die flüssige Phase, in welcher die Liposomen L dispergiert vorliegen, von der ersten Flüssigkeit 3 abgetrennt werden, was aufgrund der allenfalls sehr schlechten Mischbarkeit der hydrophilen flüssigen Phase mit der hydrophoben ersten Flüssigkeit 3 sowie deren unterschiedlicher Dichte in einfacher Weise möglich ist.

Aus den oben genannten Gründen ist es darüber hinaus möglich, dass die erste Emulsion 4 auf eine Temperatur zwischen dem Schmelzpunkt der (hydrophoben) ersten Flüssigkeit 3 und dem Schmelzpunkt der (hydrophilen) Wirkstofflösung 1 abgekühlt wird, um die Wirkstofflösung 1 der in der ersten Flüssigkeit 3 emulgierten Tropfen mit der an diese angelagerten, monomolekularen, inneren Schicht der ersten amphiphilen Verbindung 2 in den festen Zustand zu überführen, wonach die erste Emulsion 4 im festen Zustand der Tropfen der Wirkstofflösung 1 mit der (hydrophilen) flüssigen Phase der zweiten Emulsion 9, also mit deren kontinuierlicher Phase, unter Ausbildung der Phasengrenze 6 in Kontakt gebracht wird und die über die Phasengrenze 6 miteinander in Kontakt stehenden erste Emulsion 4 und die zweite Emulsion 9, insbesondere unter fortwährendem Halten der Tropfen der Wirkstofflösung 1 im festen Aggregatzustand, zentrifugiert werden.

Ferner ist es bedarfsweise beispielsweise einerseits möglich, die fertigen Liposomen L mit Polymerkonjugaten, z.B. solchen auf der Basis von Polyethylenglykol (PEG), zu modifizieren, indem sie z.B. elektrostatisch an die zweite amphiphile Verbindung 8 der äußeren Schicht der Bischicht angebunden werden (nicht gezeigt). Andererseits bietet das erfindungsgemäße Verfahren insbesondere die Möglichkeit, dass als zweite amphiphile Verbindung 8, welche die monomolekulare äußere Schicht der Bischicht der Liposomen L bildet, ein oder mehrere Lipide eingesetzt werden, wobei an zumindest einige Moleküle dieser Lipide bereits zuvor Polymerkonjugate angebunden worden sind.

Während die vorstehend unter Bezugnahme auf die Fig. 1 erläuterte Ausführungsform eines erfindungsgemäßen Verfahrens zur Verkapselung von Wirkstoffen in symmetrischen oder insbesondere asymmetrischen Liposomen L grundsätzlich, wie oben erwähnt, diskontinuierlich bzw. chargenweise oder auch semikontinuierlich durchgeführt werden können, eröffnet die Erfindung insbesondere die Möglichkeit einer kontinuierlichen und folglich im großtechnischen Maßstab besonders wirtschaftlichen Durchführung des Verfahrens.

Die Fig. 2 zeigt eine schematische Querschnittsansicht einer Ausführungsform einer zur kontinuierlichen Durchführung des Verfahrens zur Verkapselung von Wirkstoffen in Liposomen geeigneten Zentrifugiereinrichtung, wobei in der Fig. 2 aus Veranschaulichungsgründen nur der halbe Querschnitt der im Wesentlichen rotationssymmetrischen Zentrifugiereinrichtung wiedergegeben ist. Die in der Fig. 2 dargestellte, im Durchfluss betriebene, kontinuierliche Zentrifugiereinrichtung, ist um ihre Längsmittelachse 11 rotierbar, wobei das hierdurch erzeugbare Zentrifugalfeld durch die Pfeile P₃ angedeutet ist. Die Zentrifugiereinrichtung umfasst eine durch eine Umfangswand 12 begrenzte Zentrifugierkammer 13, welche sich über den Großteil ihrer axialen Länge erstreckt. An ihrer in der Fig. 2 linken Seite weist die Zentrifugiereinrichtung zwei separate Einlässe 14, 15 auf, welche beispielsweise im Wesentlichen koaxial zueinander angeordnet sind. Dabei mündet der erste Einlass 14 in einen radial äußeren Umfangsabschnitt der Zentrifugierkammer 13 ein, während der zweite Einlass 15 in einen radial inneren Zentralabschnitt der Zentrifugierkammer 13 einmündet. In dem (in der Fig. 2 linken) Einlassbereich der Zentrifugiereinrichtung befindet sich zu diesem Zweck ein sich im Wesentlichen in Radialrichtung derselben erstreckendes Einlasswehr 16, welches einerseits eine radial äu-βere, beispielsweise etwa ringförmige Durchgangsöffnung zwischen dem ersten Einlass 14 und dem radial äußeren Abschnitt der Zentrifugierkammer 13, andererseits eine radial innere, beispielsweise etwa kreisrunde Durchgangsöffnung zwischen dem zweiten Einlass 15 und dem radial inneren Abschnitt der Zentrifugierkammer 13 freilässt, so dass dem ersten Einlass 14 und dem zweiten Einlass 15 gleichzeitig aufgegebene fluide Medien mittels des Einlasswehres 16 zunächst voneinander getrennt gehalten werden, wonach sie infolge des Passierens des Einlasswehres 16 einerseits radial außen- und andererseits radial innenseitig desselben in die gemeinsame Zentrifugierkammer 13 überführt werden, und zwar einerseits in deren radial äußeren Abschnitt und andererseits in deren radial inneren Abschnitt.

An ihrer in der Fig. 2 rechten Seite weist die Zentrifugiereinrichtung zwei separate Auslässe 17, 18 auf, welche beispielsweise wiederum im Wesentlichen koaxial zueinander angeordnet sind. Dabei mündet der erste Auslass 17 aus dem radial äußeren Umfangsabschnitt der Zentrifugierkammer 13 aus, während der zweite Auslass 18 aus dem radial inneren Zentralabschnitt der Zentrifugierkammer 13 ausmündet. In dem (in der Fig. 2 rechten) Auslassbereich der Zentrifugiereinrichtung befindet sich zu diesem Zweck ein sich im Wesentlichen in Radialrichtung derselben erstreckendes Auslasswehr 19, welches einerseits eine radial äußere, beispielsweise etwa ringförmige Durchgangsöffnung zwischen dem ersten Auslass 17 und dem radial äußeren Abschnitt der Zentrifugierkammer 13, andererseits eine radial innere, beispielsweise etwa kreisrunde Durchgangsöffnung zwischen dem zweiten Auslass 18 und dem radial inneren Abschnitt der Zentrifugierkammer 13 freilässt, so dass zwei in der Zentrifugierkammer 13 zentrifugierte fluide Medien unterschiedlicher Dichte, welche insbesondere nicht oder nur schlecht miteinander mischbar sind, nach Passieren des Auslasswehres 19 einerseits radial innen- und andererseits radial außenseitig desselben voneinander getrennt werden, wonach sie einerseits in den zweiten Auslass 18, andererseits in den ersten Auslass 17 überführt werden, um sie der Zentrifugiereinrichtung zu entnehmen.

In der Zentrifugierkammer 13 befindet sich im Bereich von deren auslassseitigem Abschnitt, aber stromauf des Auslasswehres 19, ferner ein sich im Wesentlichen in Radialrichtung derselben erstreckendes erstes Rückhaltewehr 20, welches z.B. im Wesentlichen kreisringförmig ausgestaltet ist und sich von der äußeren Umfangswand 12 der Zentrifugiereinrichtung um einen radialen Abstand R₁ nach innen erstreckt, wobei dieser radiale Abstand R₁, also die radiale Breite des ersten Rückhaltewehres 20, zweckmäßigerweise wenigstens der radialen Breite der Durchgangsöffnung zwischen dem radial äußeren Ende des Auslasswehres 19 und der Umfangswand 12 entspricht bzw. diese vorzugsweise zumindest geringfügig übertrifft. Darüber hinaus befindet sich in der Zentrifugierkammer 13 im Bereich von deren auslassseitigem Abschnitt, aber wiederum stromauf des Auslasswehres 19, ein sich gleichfalls im Wesentlichen in Radialrichtung derselben erstreckendes zweites Rückhaltewehr 21, welches im vorliegenden Fall z.B. im Wesentlichen kreisringförmig ausgestaltet ist und sich zwischen einem radial etwa mittleren Abschnitt der Zentrifugierkammer 13 bis nahe der zentralen Drehachse 11 der Zentrifugiereinrichtung erstreckt, wobei es stattdessen aber auch im Wesentlichen kreisförmig ausgestaltet sein und folglich keinen zentralen Durchlasse besitzen kann (nicht gezeigt). Der radiale Abstand R₂ des zweiten Rückhaltewehres von der zentralen Drehachse 11, also die radiale Breite des zweiten Rückhaltewehres 21, entspricht zweckmäßigerweise wenigstens der radialen Breite der Durchgangsöffnung zwischen dem radial inneren Ende des Auslasswehres 19 und der zentralen Drehachse 11 bzw. übertrifft diese vorzugsweise zumindest geringfügig.

Zur kontinuierlichen Durchführung der oben unter Bezugnahme auf die Fig. 1 beschriebenen Ausführungsform des erfindungsgemäßen Verfahrens wird nun einerseits die erste Emulsion 4 gemäß Schritt (b), deren hydrophobe erste Flüssigkeit eine höhere Dichte aufweist als die hydrophile kontinuierliche Phase der zweiten Emulsion 9 (vgl. die Fig. 1), dem ersten (radial äußeren) Einlass 14 der Zentrifugiereinrichtung kontinuierlich aufgegeben; andererseits wird die zweite Emulsion 9 (vgl. die Fig. 1) dem zweiten (radial inneren) Einlass 15 der Zentrifugiereinrichtung kontinuierlich aufgegeben. Hierbei werden einerseits die erste Emulsion 4, andererseits die zweite Emulsion 9 im Einlassbereich der Zentrifugiereinrichtung durch das Einlasswehr 16 zunächst voneinander getrennt gehalten und gelangen anschließend, sobald sie das Einlasswehr 16 einerseits durch seine radial äußere Durchgangsöffnung, andererseits durch sein radial innere Durchgangsöffnung passiert haben, einerseits in den radial äußeren Abschnitt, andererseits in den radial inneren Abschnitt der gemeinsamen Zentrifugierkammer 13, womit sie gemäß Schritt (d) miteinander in Kontakt gebracht werden und sich die Phasengrenze 6 ausbildet. Eine weitere Funktion des unter- bzw. überströmbaren Einlasswehres 16 besteht dabei darin, den (radial äußeren) Einlassbereich der ersten Emulsion 4 in die Zentrifugierkammer 13 nicht mit der hydrophilen flüssigen Phase der zweiten Emulsion 9 mit demgegenüber geringerer Dichte zu überschichten, damit in diesem Einlassbereich keine unkontrollierten Bischichten der in der ersten Emulsion 4 enthaltenen Wirkstofflösungstropfen 1 mit der hieran angelagerten monomolekularen Schicht der ersten amphiphilen Verbindung 2 (vgl. die Fig. 1) erzeugt werden.

In der Zentrifugierkammer 13, welche in axialer Richtung, d.h. in der Fig. 2 von links nach rechts, durchströmt wird, findet ferner der oben unter Bezugnahme auf die Fig. 1 im Einzelnen erläuterte Zentrifugierschritt (e) statt, wobei die in der ersten Flüssigkeit 3 emulgierten Tropfen der Wirkstofflösung 1 mit der an diese angelagerten monomolekularen (inneren) Schicht der ersten amphiphilen Verbindung 2 der ersten Emulsion 4 (vgl. auch die Fig. 1) in der Zentrifugierkammer 13 mittels des ersten Rückhaltewehres 20 akkumuliert werden können. Eine Funktion des ersten Rückhaltewehres 20 besteht folglich auch darin, dass die emulgierten und mit einer Monoschicht der ersten amphiphilen Verbindung 2 versehenen Tropfen der Wirkstofflösung 1 in der ersten Emulsion 4, welche noch nicht zu einer mit einer Bischicht versehenen Liposomen L umgesetzt worden sind, nicht über den ersten Auslass 17 aus der Zentrifugierkammer 13 mitgerissen werden, sondern durch Auftriebskräfte zuvor die Phasengrenze 6 erreichen, um dort möglichst vollständig zu den Liposomen L mit einer Bischicht umgesetzt werden zu können.

Darüber hinaus können die in der hydrophilen flüssigen Phase der zweiten Emulsion 9 emulgierten Tropfen aus der hydrophoben zweiten Flüssigkeit 10, welche mit einer monomolekularen Schicht der zweiten amphiphilen Verbindung 8 versehen sind, mittels des zweiten Rückhaltewehres 21 in der Zentrifugierkammer 13 akkumuliert werden. Eine Funktion des zweiten Rückhaltewehres 21 besteht folglich vornehmlich darin, dass die emulgierten und mit einer Monoschicht der zweiten amphiphilen Verbindung 8 versehenen Tropfen der hydrophoben zweiten Flüssigkeit 10 in der zweiten Emulsion 9, welche die zweite amphiphile Verbindung 8 noch nicht an der Phasengrenze 6 abgeschieden haben, nicht über den zweiten Auslass 18 aus der Zentrifugierkammer 13 mitgerissen werden, sondern durch die induzierten Zentrifugalkräfte zuvor die Phasengrenze 6 erreichen, um dort möglichst vollständig die zweite amphiphile Verbindung 8 anzureichern.

Schließlich wird einerseits die kontinuierliche, hydrophile flüssige Phase der zweiten Emulsion 9 mit den mit der Bischicht aus der ersten amphiphilen Verbindung 2 (innere Schicht) und der zweiten amphiphilen Verbindung 8 (äußere Schicht) versehenen Liposomen L (vgl. die Fig. 1), andererseits die hydrophobe erste Flüssigkeit 3 aus der gemeinsamen Zentrifugierkammer 13 mittels des Auslasswehres 19 voneinander getrennt und einerseits über den zweiten Auslass 18, andererseits über den ersten Auslass 17 der Zentrifugiereinrichtung entnommen. In der Fig. 2 sind dabei die mittels der unter- bzw. überströmbaren Wehre eingestellten Flüssigkeitsfüllstände im Innern der Zentrifugierkammer 13 erkennbar. Dabei fließt die "leichtere" Phase mit geringerer Dichte - hier: die kontinuierliche, hydrophile flüssige Phase der zweiten Emulsion 9 (vgl. die Fig. 1) - nur dann über das Auslasswehr 19 ab, wenn die Flüssigkeitssäule beider Phasen 9 und 4 bis zum radial inneren Ende des Auslasswehres 19 denselben hydrostatischen Druck erzeugt wie die Flüssigkeitssäule der "schweren" Phase mit demgegenüber höherer Dichte - hier: der hydrophoben ersten Flüssigkeit 3 der ersten Emulsion 4 - bis zum radial inneren Ende 17a des ersten Auslasses 17. Eine Funktion des über- bzw. unterströmbaren Auslasswehres 19 besteht somit vornehmlich darin, für eine vollständige Trennung der Phasen 9 und 4 zu sorgen, wobei der Abstand der Phasengrenze 6 von dem radial äußeren Ende des Auslasswehres 19 mit Vorteil derart eingestellt werden sollte, dass die Phasengrenze 6 auch bei Schwingungen das radial äußere Ende des Auslasswehres 19 nicht zu erreichen vermag.

Nachstehend ist die Erfindung anhand von Ausführungsbeispielen näher erläutert.

| **Beispiel 1:** | Herstellung asymmetrischer Liposomen aus 10 µg/ml Viscumin (Mistelextrakt) in 15 mM Phosphatpuffer in einer Bischicht aus einerseits einer Mischung aus Ei-Lecithin mit einem Phosphatidylcholin-Gehalt von 80% (E80) und Cholesterol (Cholesterin) in einem Molverhältnis von 60:40 (innere monomolekulare Schicht) und andererseits einer Mischung aus Ei-Lecithin mit einem Phosphatidylcholin-Gehalt von 80% (E80) und Cholesterol in einem Molverhältnis von 80:20 (äußere monomolekulare Schicht). |
|---|---|
| Wirkstofflösung: | 10 µg/ml Viscumin (Wirkstoff) in 15 mM wässrigem Phosphatpuffer (PP) (hydrophiles Lösungsmittel), |
| Erste amphiphile Verbindung: | Ei-Lecithin mit einem Phosphatidylcholin-Gehalt von 80% (E80) und Cholesterol im Verhältnis von 60:40 mol-%, |
| Zweite amphiphile Verbindung: | Ei-Lecithin mit einem Phosphatidylcholin-Gehalt von 80% (E80) und Cholesterol im Verhältnis von 80:20 mol-%, |
| Erste Flüssigkeit (hydrophob): | Perfluoroperhydrophenanthren (C₁₄F₂₄, CAS-Nummer 306-91-2), |
| Zweite Flüssigkeit (hydrophob): | Perfluoroperhydrophenanthren (C₁₄F₂₄, CAS-Nummer 306-91-2), |
| Flüssige Phase (hydrophil): | 15 mM wässriger Phosphatpuffer (PP), |
| Erste Emulsion: | 1% (v/v) Wirkstofflösung (Viscumin in 15 mM wässrigem PP) in Perfluoroperhydrophenanthren mit 1,5 mM Gesamtanteil der ersten amphiphilen Verbindung (E80 und Cholesterol im Verhältnis von 60:40 mol-%), |
| Zweite Emulsion: | 1% (v/v) Perfluoroperhydrophenanthren in der hydrophilen flüssigen Phase (15 mM wässriger PP) mit 1,5 mM Gesamtanteil der zweiten amphiphilen Verbindung (E80 und Cholesterol im Verhältnis von 80:20 mol-%). |

### (a) Bereitstellen der Wirkstofflösung:

Der Wirkstoff Viscumin wird mit einem Anteil von 10 µg/ml in 15 mM wässrigem Phosphatpuffer (PP) als hydrophilem Lösungsmittel gelöst, um die hydrophile Wirkstofflösung bereitzustellen.

### (b) Bereitstellen der ersten Emulsion:

150 mM einer Mischung aus Ei-Lecithin mit einem Phosphatidylcholin-Gehalt von 80% (E80) und Cholesterol in einem molaren Verhältnis von 60:40 mol-% als erste amphiphile Verbindung werden in einem Rundkolben in Ethanol gelöst, wonach das Ethanol mittels eines Rotationsverdampfers und anschließender Einlagerung des Rundkolbens in einem Exsikkator vollständig verdampft wird, so dass das E80/Cholesterol im Molverhältnis von 60:40 mol-% als trockener Film in dem Rundkolben zurückbleibt. Die hydrophile Wirkstofflösung aus 10 µg/ml in 15 mM PP gemäß Schritt (a) wird nun dem Rundkolben mit dem E80/Cholesterol (60:40 mol-%) zugesetzt, um eine Mischung aus 150 mM E80/Cholesterol (60:40 mol-%) in der Wirkstofflösung zu erhalten. Das E80/Cholesterol (60:40 mol-%) (erste amphiphile Verbindung) löst sich hierbei in der hydrophilen Wirkstofflösung infolge Quellung von der Glaswand des Rundkolbens ab und bildet polydisperse amphiphile Aggregate, welche durch mechanische Bearbeitung zerkleinert werden. Hierzu werden zwei alternative Methoden angewandt:
- Extrusion durch 800 nm und 400 nm Poren; und
- Anwendung von Ultraschall (*Hielscher UP 2005* mit 200 W Maximalleistung und einer Ultraschallfrequenz von 26 kHz): 10 Sekunden mit einem Anteil von 100% des Beschallungszyklus (Zyklusdauer: je 1 Sekunde) und einer Amplitude von 50%; auf die initiale Beschallung der ersten 10 Sekunden folgt eine Beschallung über 10 Minuten mit einem Zyklusanteil von 50% und einer Amplitude von 40% ohne Kühlung zur Zerkleinerung der amphiphilen Aggregate auf eine mittlere Partikelgröße von 276 nm ±50 nm.

Die auf diese Weise erzeugte Mischung der Wirkstofflösung mit den suspendierten Aggregaten aus E80/Cholesterol (60:40 mol-%) (erste amphiphile Verbindung) wird mit einem Volumenanteil von 1% in Perfluoroperhydrophenanthren (erste Flüssigkeit, hydrophob) eingebracht. Zur Emulgierung wird die Wirkstofflösung mit dem E80/ Cholesterol (60:40 mol-%) als disperse Phase mittels Ultraschall (*Hielscher UP 2005*) für 10 Sekunden mit ei-nem Zyklusanteil von 100% und einer Amplitude von 50%, gefolgt von 10 Minuten mit einem Zyklusanteil von 50% und einer Amplitude von 40%, ohne Kühlung suspendiert, so dass die erste Emulsion aus 1% (v/v) hydrophiler Wirkstofflösung (Viscumin in 15 mM wässrigem PP) in der ersten hydrophoben Flüssigkeit (Perfluoroperhydrophenanthren) mit 1,5 mM Gesamtanteil der ersten amphiphilen Verbindung (E80 und Cholesterol im Verhältnis von 60:40 mol-%) besteht.

Auf diese Weise werden Pre-Liposomen aus den Wirkstofflösungstropfen mit einer monomolekularen Schicht aus E80/Cholesterol im Molverhältnis von 60:40 mol-% (disperse Phase der ersten Emulsion) in Perfluoroperhydrophenanthren (erste Flüssigkeit, hydrophob als kontinuierliche Phase der ersten Emulsion) mit einer mittleren Partikelgröße von 376 nm ± 22 nm, einer Partikelanzahl von 482 kcounts/s ± 35 kcounts/s (derived count rate) und einem Polydispersitätsindex (PDI) von 1,0 erzeugt.

Unmittelbar anschließend wird die erste Emulsion mit den Pre-Liposomen aliquotiert, wobei sie für die nachfolgende kontinuierliche Zentrifugation (siehe Schritt (e) weiter unten) beispielsweise auf Volumina von 30 ml aliquotiert wird.

### (c) Bereitstellen der Mischung - hier: in Form der zweiten Emulsion - aus der mit der ersten Flüssigkeit (Perfluoroperhydrophenanthren, hydrophob) der ersten Emulsion nicht oder schlecht mischbaren flüssigen Phase (hydrophil) mit der zweiten amphiphilen Verbindung mit hierin emulgierter zweiter Flüssigkeit (hydrophob):

E80/Cholesterol im Molverhältnis von 80:20 mol-% als zweite amphiphile Verbindung wird in einem Rundkolben in Ethanol gelöst, wonach das Ethanol mittels eines Rotationsverdampfers und anschließender Einlagerung des Rundkolbens in einem Exsikkator vollständig verdampft wird, so dass die zweite amphiphile Verbindung als trockener Film in dem Rundkolben zurückbleibt.

Die hydrophile flüssige Phase aus 15 mM wässrigem PP wird nun dem Rundkolben mit dem E80/Cholesterol im Molverhältnis von 80:20 mol-% zugesetzt, um eine Mischung aus 150 mM E80/Cholesterol im Molverhältnis von 80:20 mol-% (zweite amphiphile Verbindung) in 15 mM wässrigem PP (hydrophile flüssige Phase) zu erhalten. Das E80/ Cholesterol im Molverhältnis von 80:20 mol-% löst sich hierbei in der hydrophilen Wirkstofflösung infolge Quellung von der Glaswand des Rundkolbens ab und bildet polydisperse amphiphile Aggregate, welche durch mechanische Bearbeitung zerkleinert werden. Hierzu können wiederum 2 alternative Methoden angewandt werden:
- Extrusion durch 800 nm und 400 nm Poren; und
- Anwendung von Ultraschall (*Hielscher UP 2005* mit 200 W Maximalleistung und einer Ultraschallfrequenz von 26 kHz): 10 Sekunden mit einem Anteil von 100% des Beschallungszyklus (Zyklusdauer: je 1 Sekunde) und einer Amplitude von 50%; auf die initiale Beschallung der ersten 10 Sekunden folgt eine Beschallung über 10 Minuten mit einem Zyklusanteil von 50% und einer Amplitude von 40% ohne Kühlung zur Zerkleinerung der amphiphilen Aggregate.

In die auf diese Weise erzeugte Mischung der hydrophilen flüssigen Phase (15 mM wässriger PP) mit den suspendierten Aggregaten aus E80/Cholesterol im Molverhältnis von 80:20 mol-% (zweite amphiphile Verbindung) wird nun zur Erzeugung der zweiten Emulsion die hydrophobe zweite Flüssigkeit (Perfluoroperhydrophenanthren) mit einem Volumenanteil von etwa 1% eindispergiert. Zur Emulgierung wird das Perfluoroperhydrophenanthren als disperse Phase mittels Ultraschall *(Hielscher UP 2005*) für 10 Sekunden mit einem Zyklusanteil von 100% und einer Amplitude von 50%, gefolgt von 30 Minuten mit einem Zyklusanteil von 75% und einer Amplitude von 60%, ohne Kühlung suspendiert, so dass die zweite Emulsion aus 1% (v/v) hydrophober zweiter Flüssigkeit (Perfluoroperhydrophenanthren) in der hydrophilen flüssigen Phase (15 mM wässriger PP) mit 1,5 mM Gesamtanteil der zweiten amphiphilen Verbindung (E80 und Cholesterol im Verhältnis von 80:20 mol-%) besteht.

Auf diese Weise werden Amphiphil-Träger aus den Perfluoroperhydrophenanthren-Tropfen mit einer monomolekularen Schicht aus der zweiten amphiphilen Verbindung E80/Cholesterol im Molverhältnis von 80:20 mol-% (disperse Phase der zweiten Emulsion) in 15 mM wässrigem PP (flüssige Phase, hydrophil als kontinuierliche Phase der zweiten Emulsion) mit einer mittleren Partikelgröße von 127 nm ± 6 nm, einer Partikelanzahl von 1411 kcounts/s ± 52 kcounts/s (derived count rate) und einem Polydispersitätsindex (PDI) von 0,298 erzeugt.

Unmittelbar anschließend wird auch die zweite Emulsion mit den Amphiphil-Trägern auf Volumina von 30 ml aliquotiert.

### (d) Inkontaktbringen der ersten Emulsion (kontinuierliche Phase aus der ersten Flüssigkeit Perfluoroperhydrophenanthren, hydrophob; disperse Phase aus Pre-Liposomen aus der hydrophilen Wirkstofflösung mit monomolekularer Schicht aus E80/Cholesterol im Molverhältnis von 60:40 mol-%) mit der zweiten Emulsion (kontinuierliche Phase aus der flüssigen Phase 15 mM PP in Wasser, hydrophil; disperse Phase aus Amphiphil-Trägern aus der hydrophoben zweiten Flüssigkeit Perfluoroperhydrophenanthren mit monomolekularer Schicht aus E80/Cholesterol im Molverhältnis von 80:20 mol-%):

Die gemäß dem obigen Schritt (b) aliquotierten Volumina von 30 ml der ersten Emulsion mit den Pre-Liposomen werden ungekühlt bei Raumtemperatur verwendet und gemäß dem nachfolgenden Schritt (e) in einer im Durchfluss betriebenen, kontinuierlichen Zentrifugiereinrichtung, wie sie oben unter Bezugnahme auf die Fig. 2 im Einzelnen erläutert ist, mit den gemäß dem obigen Schritt (c) aliquotierten Volumina von 30 ml der zweiten Emulsion mit den Amphiphil-Trägern in Kontakt gebracht, wobei sich eine Phasengrenze zwischen den kontinuierlichen Phasen der ersten und zweiten Emulsion ausbildet, an welcher sich die Mischung aus E80 und Cholesterol anreichern kann, ohne ein Organogel zu bilden.

### (e) Zentrifugieren der ersten Emulsion (kontinuierliche Phase aus der ersten Flüssigkeit Perfluoroperhydrophenanthren, hydrophob; disperse Phase aus Pre-Liposomen aus der hydrophilen Wirkstofflösung mit monomolekularer Schicht aus E80/Cholesterol im Molverhältnis von 60:40 mol-%) mit der hiermit über die Phasengrenze in Kontakt stehenden zweiten Emulsion (kontinuierliche Phase aus der flüssigen Phase 15 mM PP in Wasser, hydrophil; disperse Phase aus Amphiphil-Trägern aus der hydrophoben zweiten Flüssigkeit Perfluoroperhydrophenanthren mit monomolekularer Schicht aus E80/Cholesterol im Molverhältnis von 80:20 mol-%):

Zunächst wird das Totvolumen der kontinuierlichen Zentrifuge bei 1000 g (entsprechend etwa 9800 m/s²) durch kontinuierliche Injektion von ca. 13 ml einerseits der ersten Emulsion (hydrophobe erste Flüssigkeit Perfluoroperhydrophenanthren mit den hierin suspendierten Pre-Liposomen) mit einer Flussrate von ca. 1 ml/min, andererseits der zweiten Emulsion (hydrophile flüssige Phase 15 mM PP in Wasser mit den hierin suspendierten Amphiphil-Trägern) befüllt. Ab dem Überlaufen der Zentrifugierkammer werden beide Phasen (hydrophil/hydrophob) mit einer Flussrate von ca. 0,3 ml/min kontinuierlich zugeführt und weiterhin mit 1000 g kontinuierlich zentrifugiert. Hierbei werden einerseits die Amphiphil-Träger aus der zweiten Emulsion im Zentrifugalfeld kontinuierlich der Phasengrenze zugeführt, um die zweite amphiphile Verbindung (E80/Cholesterol im Molverhältnis von 80:20 mol-%) fortwährend an der Phasengrenze anzureichern. Andererseits werden die Pre-Liposomen aus der ersten Emulsion bzw. aus deren ersten Flüssigkeit (Perfluoroperhydrophenanthren) durch die Phasengrenze hindurch in die flüssige Phase (15 mM PP in Wasser) überführt, wobei sich die an der Phasengrenze angereicherte zweite amphiphile Verbindung (E80/Cholesterol im Molverhältnis von 80:20 mol-%) als zweite (äußere) monomolekulare Schicht an die erste (innere) monomolekulare Schicht aus der ersten amphiphilen Verbindung (E80/Cholesterol im Molverhältnis von 60:40) anlagert, um die fertigen Liposomen zu bilden. Die Gesamtvolumina beider Phasen (hydrophil/hydrophob) betragen dabei ca. 30 ml. Nach Abschluss der kontinuierlichen Zuführung der beiden Phasen (hydrophil/hydrophob) nach ca. 70 min. werden die beiden Phasen noch für weitere 20 Minuten bei 1000 g zentrifugiert. Die über die Auslasswehre (vgl. die Fig. 2) abfließenden Phasen werden gesammelt.

Die hydrophile flüssige Phase (15 mM PP in Wasser) mit den hierin suspendierten fertigen Liposomen wird mittels Photonenkorrelationsspektroskopie (PCS, *Malvern Zetasizer Nano Z590)* analysiert, um die Größe der derart erzeugten Liposomen zu bestimmen. Die auf diese Weise erzeugten Liposomen, welche eine unterschiedliche Zusammensetzung der inneren und äußeren monomolekularen Schicht ihrer Bischicht besitzen, weisen eine Partikelgröße von 367 nm ± 61 nm, eine Partikelanzahl von 3207 ± 52 kcounts/s (derived count rate) sowie einen Polydispersitätsindex (PDI) von 0,30 auf.

| **Beispiel 2:** | Herstellung asymmetrischer Liposomen aus Pyranin-(8-hydroxy-1,3,5-pyrentrisulfonsäuretrinatriumsalz (HPTS) in 150 mM Phosphatpuffer in einer Bischicht aus einerseits Dipalmitoylphosphatidylcholin (DPPC, innere monomolekulare Schicht) und andererseits Distearoylphosphatidylcholin (DSPC, äußere monomolekulare Schicht). |
|---|---|
| Wirkstoffmarkerlösung: | Pyranin-(8-hydroxy-1,3,5-pyrentri-sulfonsäuretrinatriumsalz (HPTS) (Surrogat) in 150 mM wässrigem Phosphatpuffer (PP) (hydrophiles Lösungsmittel), |
| Erste amphiphile Verbindung: | Dipalmitoylphosphatidylcholin (DPPC), |
| Zweite amphiphile Verbindung: | Distearotoylphosphatidylcholin (DSPC), |
| Erste Flüssigkeit (hydrophob): | Squalen (2,6,10,15,19,23-Hexamethyl-2,6,10,14,18,22-tetracosahexaen, C₃₀H₅₀, CAS-Nummer 111-02-4), |
| Zweite Flüssigkeit (hydrophob): | Squalen (2,6,10,15,19,23-Hexamethyl-2,6,10,14,18,22-tetracosahexaen, C30H50, CAS-Nummer 111-02-4), |
| Flüssige Phase (hydrophil): | 150 mM wässriger Phosphatpuffer (PP), |
| Erste Emulsion: | 0,5% (v/v) Wirkstoffmarkerlösung (HPTS in 150 mM wässrigem PP) in Squalen mit 0,27 mM Gesamtanteil der ersten amphiphilen Verbindung (DPPC), |
| Zweite Emulsion: | 10% (v/v) Squalen in der hydrophilen flüssigen Phase (150 mM wässriger PP) mit 0,27 mM Gesamtanteil der zweiten amphiphilen Verbindung (DSPC). |

### (a) Bereitstellen der Wirkstoffmarkerlösung:

Der Wirkstoffmarker HPTS (Surrogat) wird in 150 mM wässrigem Phosphatpuffer (PP) als hydrophilem Lösungsmittel gelöst, um die hydrophile Wirkstoffmarkerlösung bereitzustellen.

### (b) Bereitstellen der ersten Emulsion:

Das Dipalmitoylphosphatidylcholin (DPPC) als erste amphiphile Verbindung wird in einem Rundkolben in Ethanol gelöst, wonach das Ethanol mittels eines Rotationsverdampfers und anschließender Einlagerung des Rundkolbens in einem Exsikkator vollständig verdampft wird, so dass das DPPC als trockener Film in dem Rundkolben zurückbleibt.

Die hydrophile Wirkstoffmarkerlösung aus HPTS in 150 mM PP gemäß Schritt (a) wird nun dem Rundkolben mit dem DPPC zugesetzt, um eine Mischung aus DPPC in der Wirkstoffmarkerlösung zu erhalten. Das DPPC (erste amphiphile Verbindung) löst sich hierbei in der hydrophilen Wirkstoffmarkerlösung infolge Quellung von der Glaswand des Rundkolbens ab und bildet polydisperse amphiphile Aggregate, welche durch mechanische Bearbeitung zerkleinert werden. Die mechanische Bearbeitung geschieht beispielsweise durch Anwendung von Ultraschall (*Hielscher UP 2005* mit 200 W Maximalleistung und einer Ultraschallfrequenz von 26 kHz): 10 Sekunden mit einem Anteil von 100% des Beschallungszyklus (Zyklusdauer: je 1 Sekunde) und einer Amplitude von 50%; auf die initiale Beschallung der ersten 10 Sekunden folgt eine Beschallung über 10 Minuten mit einem Zyklusanteil von 50% und einer Amplitude von 40% mit Kühlung zur Zerkleinerung der amphiphilen Aggregate.

Die auf diese Weise erzeugte Mischung der Wirkstoffmarkerlösung mit den suspendierten Aggregaten aus DPPC (erste amphiphile Verbindung) wird mit einem Volumenanteil von 1% in Squalen (erste Flüssigkeit, hydrophob) eingebracht. Zur Emulgierung wird die Wirkstoffmarkerlösung mit dem DPPC als disperse Phase mittels Ultraschall (*Hielscher UP 2005*) für 10 Sekunden mit einem Zyklusanteil von 100% und einer Amplitude von 50%, gefolgt von 10 Minuten mit einem Zyklusanteil von 50% und einer Amplitude von 40%, mit Kühlung suspendiert. Auf diese Weise werden Pre-Liposomen aus den Wirkstoffmarkerlösungstropfen mit einer monomolekularen Schicht aus DPPC (disperse Phase der ersten Emulsion) in Squalen (erste Flüssigkeit, hydrophob als kontinuierliche Phase der ersten Emulsion) mit einer mittleren Partikelgröße von 827 nm ± 80 nm, einer Partikelanzahl von 311 kcounts/s ± 22 kcounts/s (derived count rate) und einem Polydispersitätsindex (PDI) von 0,15 erzeugt.

Unmittelbar anschließend wird die erste Emulsion mit den Pre-Liposomen aliquotiert, wobei sie für die nachfolgende kontinuierliche Zentrifugation (siehe Schritt (e) weiter unten) beispielsweise auf Volumina von 30 ml aliquotiert wird.

### (c) Bereitstellen der Mischung - hier: in Form der zweiten Emulsion - aus der mit der ersten Flüssigkeit (Squalen, hydrophob) der ersten Emulsion nicht oder schlecht mischbaren flüssigen Phase (hydrophil) mit der zweiten amphiphilen Verbindung mit hierin emulgierter zweiter Flüssigkeit (hydrophob):

Das Distearoylphosphatidylcholin (DSPC) als zweite amphiphile Verbindung wird in einem Rundkolben in Ethanol gelöst, wonach das Ethanol mittels eines Rotationsverdampfers und anschließender Einlagerung des Rundkolbens in einem Exsikkator vollständig verdampft wird, so dass die zweite amphiphile Verbindung als trockener Film in dem Rundkolben zurückbleibt.

Die hydrophile flüssige Phase aus 150 mM wässrigem PP wird nun dem Rundkolben mit dem DSPC zugesetzt, um eine Mischung aus DSPC(zweite amphiphile Verbindung) in 150 mM wässrigem PP (hydrophile flüssige Phase) zu erhalten. Das DSPC löst sich hierbei in der hydrophilen Wirkstofflösung infolge Quellung von der Glaswand des Rundkolbens ab und bildet polydisperse amphiphile Aggregate, welche durch mechanische Bearbeitung zerkleinert werden. Die mechanische Bearbeitung geschieht beispielsweise durch Anwendung von Ultraschall (*Hielscher UP 2005* mit 200 W Maximalleistung und einer Ultraschallfrequenz von 26 kHz): 10 Sekunden mit einem Anteil von 100% des Beschallungszyklus (Zyklusdauer: je 1 Sekunde) und einer Amplitude von 50%; auf die initiale Beschallung der ersten 10 Sekunden folgt eine Beschallung über 10 Minuten mit einem Zyklusanteil von 50% und einer Amplitude von 40% mit Kühlung zur Zerkleinerung der amphiphilen Aggregate.

In die auf diese Weise erzeugte Mischung der hydrophilen flüssigen Phase (150 mM wässriger PP) mit den suspendierten Aggregaten aus DSPC (zweite amphiphile Verbindung) wird nun zur Erzeugung der zweiten Emulsion die hydrophobe zweite Flüssigkeit (Squalen) mit einem Volumenanteil von etwa 1% eindispergiert. Zur Emulgierung wird das Squalen als disperse Phase mittels Ultraschall (*Hielscher UP 2005*) für 10 Sekunden mit einem Zyklusanteil von 100% und einer Amplitude von 50%, gefolgt von 10 Minuten mit einem Zyklusanteil von 50% und einer Amplitude von 40%, mit Kühlung suspendiert, so dass die zweite Emulsion aus 10% (v/v) hydrophober zweiter Flüssigkeit (Squalen) in der hydrophilen flüssigen Phase (150 mM wässriger PP) mit 0,27 mM Gesamtanteil der zweiten amphiphilen Verbindung (DSPC) besteht.

Auf diese Weise werden Amphiphil-Träger aus den Squalen-Tropfen mit einer monomolekularen Schicht aus der zweiten amphiphilen Verbindung DSPC (disperse Phase der zweiten Emulsion) in 150 mM wässrigem PP (flüssige Phase, hydrophil als kontinuierliche Phase der zweiten Emulsion) mit einer mittleren Partikelgröße von 1363 nm ± 78 nm, einer Partikelanzahl von 474 kcounts/s ± 32 kcounts/s (derived count rate) und einem Polydispersitätsindex (PDI) von 0,45 erzeugt.

Unmittelbar anschließend wird auch die zweite Emulsion mit den Amphiphil-Trägern auf Volumina von 30 ml aliquotiert.

### (d) Inkontaktbringen der ersten Emulsion (kontinuierliche Phase aus der ersten Flüssigkeit Squalen, hydrophob; disperse Phase aus Pre-Liposomen aus der hydrophilen Wirkstoffmarkerlösung mit monomolekularer Schicht aus DPPC) mit der zweiten Emulsion (kontinuierliche Phase aus der flüssigen Phase 150 mM PP in Wasser, hydrophil; disperse Phase aus Amphiphil-Trägern aus der hydrophoben zweiten Flüssigkeit Squalen mit monomolekularer Schicht aus DSPC):

Die gemäß dem obigen Schritt (b) aliquotierten Volumina von 30 ml der ersten Emulsion mit den Pre-Liposomen werden ungekühlt bei Raumtemperatur verwendet und gemäß dem nachfolgenden Schritt (e) in einer im Durchfluss betriebenen, kontinuierlichen Zentrifugiereinrichtung, wie sie oben unter Bezugnahme auf die Fig. 2 im Einzelnen erläutert ist, mit den gemäß dem obigen Schritt (c) aliquotierten Volumina von 30 ml der zweiten Emulsion mit den Amphiphil-Trägern in Kontakt gebracht, wobei sich eine Phasengrenze zwischen den kontinuierlichen Phasen der ersten und zweiten Emulsion ausbildet, an welcher sich das DSPC anreichern kann.

### (e) Zentrifugieren der ersten Emulsion (kontinuierliche Phase aus der ersten Flüssigkeit Squalen, hydrophob; disperse Phase aus Pre-Liposomen aus der hydrophilen Wirkstoffmarkerlösung mit monomolekularer Schicht aus DPPC) mit der hiermit über die Phasengrenze in Kontakt stehenden zweiten Emulsion (kontinuierliche Phase aus der flüssigen Phase 150 mM PP in Wasser, hydrophil; disperse Phase aus Amphiphil-Trägern aus der hydrophoben zweiten Flüssigkeit Squalen mit monomolekularer Schicht aus DSPC):

Zunächst wird das Totvolumen der kontinuierlichen Zentrifuge bei 1000 g (entsprechend etwa 9800 m/s²) durch kontinuierliche Injektion von ca. 13 ml einerseits der ersten Emulsion (hydrophobe erste Flüssigkeit Squalen mit den hierin suspendierten Pre-Liposomen) mit einer Flussrate von ca. 1 ml/min, andererseits der zweiten Emulsion (hydrophile flüssige Phase 150 mM PP in Wasser mit den hierin suspendierten Amphiphil-Trägern) befüllt. Ab dem Überlaufen der Zentrifugierkammer werden beide Phasen (hydrophil/hydrophob) mit einer Flussrate von ca. 0,3 ml/min kontinuierlich zugeführt und weiterhin mit 1000 g kontinuierlich zentrifugiert. Hierbei werden einerseits die Amphiphil-Träger aus der zweiten Emulsion im Zentrifugalfeld kontinuierlich der Phasengrenze zugeführt, um die zweite amphiphile Verbindung (DSPC) fortwährend an der Phasengrenze anzureichern. Andererseits werden die Pre-Liposomen aus der ersten Emulsion bzw. aus deren ersten Flüssigkeit (Squalen) durch die Phasengrenze hindurch in die flüssige Phase (150 mM PP in Wasser) überführt, wobei sich die an der Phasengrenze angereicherte zweite amphiphile Verbindung (DSPC) als zweite (äußere) monomolekulare Schicht an die erste (innere) monomolekulare Schicht aus der ersten amphiphilen Verbindung (DPPC) anlagert, um die fertigen Liposomen zu bilden. Die Gesamtvolumina beider Phasen (hydrophil/hydrophob) betragen dabei ca. 30 ml. Nach Abschluss der kontinuierlichen Zuführung der beiden Phasen (hydrophil/hydrophob) werden die beiden Phasen noch für weitere 20 Minuten bei 1000 g und Raumtemperatur zentrifugiert. Die über die Auslasswehre (vgl. die Fig. 2) abfließenden Phasen werden gesammelt.

Die hydrophile flüssige Phase (150 mM PP in Wasser) mit den hierin suspendierten fertigen - asymmetrischen - Liposomen wird analog dem Beispiel 1 mittels Photonenkorrelationsspektroskopie analysiert, um die mittlere Größe der derart erzeugten Liposomen zu bestimmen. Die auf diese Weise erzeugten Liposomen, welche eine unterschiedliche Zusammensetzung der inneren und äußeren monomolekularen Schicht ihrer Bischicht besitzen, weisen eine Partikelgröße von 221 nm ± 2 nm, eine Partikelanzahl von 97 kcounts/s ± 2 kcounts/s (derived count rate) sowie einen Polydispersitätsindex (PDI) von 0,05 auf.

### Asymmetrienachweis:

Die gemäß dem obigen Ausführungsbeispiel 2 erzeugten Liposomen wurden wie folgt auf den Asymmetriegrad der ersten und zweiten monomolekularen Schicht ihrer Bischicht untersucht:
Das Beispiel 2 wurde zum Einen in der obigen Weise durchgeführt, aber mit der Maßgabe, dass nur der für die innere monomolekulare Schicht vorgesehenen ersten amphiphilen Verbindung DPPC 1 mol-% einer Markierungssubstanz (N-(7-Nitrobenz-2-oxa-1,3-diazol-4-yl)-1,2-dihexadecanoyl-sn-glycero-3-phosphoethanolamin, NBD-PE) zugesetzt worden ist. Zum Anderen wurde das Beispiel 2 in der obigen Weise durchgeführt, aber mit der Maßgabe, dass nur der für die äußere monomolekulare Schicht vorgesehenen zweiten amphiphilen Verbindung DSPC 1 mol-% der Markierungssubstanz (NBD-PE) zugesetzt worden ist.

Die Fig. 3 zeigt ein Schaubild des Asymmetrienachweises der Liposomen, wobei auf der y-Achse die für die Konzentration der Markierungssubstanz NBD-FE repräsentative relative Signalverteilung (relative signal distribution, RSD) aufgetragen ist; der linke Balken stellt das Ergebnis des Experimentes dar, bei welchem nur die innere monomolekulare Schicht ("I") der Bischicht aus DPPC mit NBD-FE markiert worden ist ("NBD-PE (I)"), während der rechte Balken das Ergebnis des Experimentes darstellt, bei welchem nur die äußere monomolekulare Schicht ("A") der Bischicht aus DSPC mit NBD-PE markiert worden ist ("NBD-PE (A)"). Der schwarze Anteil eines jeden Balkens repräsentiert den Anteil des Signals der inneren monomolekularen Schicht der Bischicht ("Signal (I)"), während der weiße Anteil eines jeden Balkens den Anteil des Signals der äußeren monomolekularen Schicht der Bischicht ("Signal (A)") repräsentiert. Man erkennt aus der Fig. 3, dass sich bei dem Experiment mit Markierung nur der inneren Schicht (DPPC; in Fig. 3 links) eine relative Signalintensität von ca. 90% auf der Membraninnenseite ergibt, während sich bei dem Experiment mit Markierung nur der äußeren Schicht (DSPC; in Fig. 3 rechts) eine relative Signalintensität von > 85% auf der Membranaußenseite ergibt, was auf eine entsprechend hohe Asymmetrie der Liposomen mit einer Bischicht aus den Lipiden DPPC (innere Schicht) und DSPC (äußere Schicht) hinweist.

Um die Verteilung der Markierungssubstanz NBD-PE in der inneren bzw. äußeren Schicht der Bischicht der gemäß dem Ausführungsbeispiel 2 erzeugten Liposomen zu bestimmen, wurde darüber hinaus bei den beiden Experimenten, bei welchen einerseits nur die innere monomolekulare Schicht ("I") aus der ersten amphiphilen Verbindung (DPPC), andererseits nur die äußere monomolekulare Schicht ("A") aus der zweiten amphiphilen Verbindung (DSPC) mit der Markierungssubstanz NBD-PE markiert worden ist, jeweils zunächst das in der äu-ßeren Schicht der Bischicht eingelagerte NBD-PE durch Zugabe von Natriumdithionit zerstört, um das von NBD-PE in der äußeren Schicht der Bischicht der Liposomen emittierte Signal auszulöschen. Durch spätere Zugabe von Octoxinol 9 ("Detergens Triton-X"^{™}) wurde sodann die Membran der Liposomen jeweils durchlässig gemacht, so dass das Natriumdithionit auch in die innere Schicht der Bischicht eindringt und auch das dort eingelagerte NED-PE zerstört, um auch das von NBD-PE in der inneren Schicht der Bischicht der Liposomen emittierte Signal auszulöschen.

Während die Fig. 4 den Verlauf der Signalintensität ("Int.") über die Zeit ("t") bei dem Experiment zeigt, bei welchen nur die innere monomolekulare Schicht ("I") aus der ersten amphiphilen Verbindung (DPPC) markiert worden ist, wenn die Markierungssubstanz (NBD-PE) in der vorstehend beschriebenen Weise zerstört wird, zeigt die Fig. 5 den Verlauf der Signalintensität ("Int.") über die Zeit ("t") bei dem Experiment, bei welchen nur die äußere monomolekulare Schicht ("A") aus der zweiten amphiphilen Verbindung (DSPC) markiert worden ist, wenn die Markierungssubstanz (NBD-PE) in der vorstehend beschriebenen Weise zerstört wird. Auch aus den Fig. 4 und 5 wird deutlich, dass die Intensität der von NBD-PE emittierten Signale um ein Vielfaches stärker abfällt, wenn das NPD-PE in der jeweils markierten Schicht (in Fig. 4 die innere Schicht "NBD-PE (I)" aus DPPC und in Fig. 5 die äußere Schicht "NBD-PE (A)" aus DSPC) zerstört wird, was darauf hinweist, dass in der jeweils nicht mit NBD-PE markierten Schicht (in Fig. 4 die äußere Schicht aus DSPC und in Fig. 5 die innere Schicht aus DSPC) nur ein sehr geringer Anteil des jeweils nicht markierten Lipides vorhanden ist, was wiederum auf eine sehr hohe Asymmetrie der Liposomen schließen lässt.

## Patentansprüche

1. Verfahren zur Verkapselung von Wirkstoffen in Liposomen (L), umfassend
- eine, insbesondere hydrophile, Lösung (1) des Wirkstoffes und
- wenigstens eine Bischicht aus zwei zumindest monomolekularen Schichten aus wenigstens einer ersten amphiphilen Verbindung (2) und wenigstens einer zweiten amphiphilen Verbindung (8), insbesondere jeweils aus der Gruppe der Lipide,
wobei die Wirkstofflösung (1) durch die Bischicht verkapselt ist, umfassend die folgenden Schritte:
(a) Bereitstellen einer, insbesondere hydrophilen, Wirkstofflösung (1) des zu verkapselnden Wirkstoffes, indem der Wirkstoff in wenigstens einem, insbesondere hydrophilen, Lösungsmittel gelöst wird;
(b) Bereitstellen einer ersten Emulsion (4) durch Emulgieren der Wirkstofflösung (1) gemäß Schritt (a) in wenigstens einer mit dem wenigstens einen Lösungsmittel der Wirkstofflösung (1) nicht oder schlecht mischbaren, insbesondere hydrophoben, ersten Flüssigkeit (3) in Gegenwart der wenigstens einen ersten amphiphilen Verbindung (2), um eine zumindest monomolekulare, innere Schicht der wenigstens einen ersten amphiphilen Verbindung (2) an die in der ersten Flüssigkeit (3) emulgierten Tropfen der Wirkstofflösung (1) anzulagern;
(c) Bereitstellen einer Mischung (7) aus einer mit der ersten Flüssigkeit (3) der ersten Emulsion (4) gemäß Schritt (b) nicht oder schlecht mischbaren, insbesondere hydrophilen, flüssigen Phase mit der wenigstens einen zweiten amphiphilen Verbindung (8) ;
(d) Inkontaktbringen der ersten Emulsion (4) aus der ersten Flüssigkeit (3) mit den hierin emulgierten Tropfen der Wirkstofflösung (1) mit der hieran angelagerten, zumindest monomolekularen, inneren Schicht der wenigstens einen ersten amphiphilen Verbindung (2) gemäß Schritt (b) mit der Mischung (7) aus der flüssigen Phase mit der wenigstens einen zweiten amphiphilen Verbindung (8) gemäß Schritt (c) unter Ausbildung einer Phasengrenze (6) zwischen der ersten Emulsion (4) gemäß Schritt (b) und der Mischung (7) gemäß Schritt (c), wobei die wenigstens eine zweite amphiphile Verbindung (8) an der Phasengrenze (6) angereichert wird; und
(e) Zentrifugieren der über die Phasengrenze (6) miteinander in Kontakt stehenden ersten Emulsion (4) gemäß Schritt (b) und der Mischung (7) gemäß Schritt (c), um die Tropfen der in der ersten Emulsion (4) enthaltenen Wirkstofflösung (1) mit der hieran angelagerten, zumindest monomolekularen, inneren Schicht der wenigstens einen ersten amphiphilen Verbindung (2) aus der ersten Flüssigkeit (3) der ersten Emulsion (4) gemäß Schritt (b) in die flüssige Phase der Mischung (7) gemäß Schritt (c) zu überführen, wobei beim Passieren der Phasengrenze (6) die dort angereicherte, wenigstens eine zweite amphiphile Verbindung (8) unter Bildung einer zumindest monomolekularen, äußeren Schicht derselben an die zumindest monomolekulare innere Schicht der wenigstens einen ersten amphiphilen Verbindung (2) der Tropfen der Wirkstofflösung (1) angelagert wird, um die Bischicht aus den beiden zumindest monomolekularen Schichten der wenigstens einen ersten amphiphilen Verbindung (2) und der wenigstens einen zweiten amphiphilen Verbindung (8) zu erzeugen.

2. Verfahren nach Anspruch 1, **dadurch gekennzeichnet, dass**
- als Mischung (7) aus der, insbesondere hydrophilen, flüssigen Phase mit der wenigstens einen zweiten amphiphilen Verbindung (8) gemäß Schritt (c) eine zweite Emulsion (9) aus der flüssigen Phase und wenigstens einer hiermit nicht oder schlecht mischbaren, insbesondere hydrophoben, zweiten Flüssigkeit (10) mit der wenigstens einen zweiten amphiphilen Verbindung (8) eingesetzt wird, indem die zweite Flüssigkeit (10) in der flüssigen Phase in Gegenwart der wenigstens einen zweiten amphiphilen Verbindung (8) emulgiert wird, um eine zumindest monomolekulare Schicht der wenigstens einen zweiten amphiphilen Verbindung (8) an die in der flüssigen Phase emulgierten Tropfen der zweiten Flüssigkeit (10) anzulagern und die zweite amphiphile Verbindung (8) auf diese Weise an diesen Tropfen zu immobilisieren; und
- beim Zentrifugieren der über die Phasengrenze (6) miteinander in Kontakt stehenden ersten Emulsion (4) gemäß Schritt (b) und der in Form der zweiten Emulsion (9) vorliegenden Mischung (7) gemäß Schritt (c) die Tropfen der zweiten Flüssigkeit (10) mit der hieran angelagerten, zumindest monomolekularen Schicht der wenigstens einen zweiten amphiphilen Verbindung (8) aus der flüssigen Phase der zweiten Emulsion (9) fortwährend an die Phasengrenze (6) zwischen der ersten Emulsion (4) und der zweiten Emulsion (9) überführt werden, um die wenigstens eine zweite amphiphile Verbindung (8) fortwährend an der Phasengrenze (6) anzureichern.

3. Verfahren nach Anspruch 1 oder 2, **dadurch gekennzeichnet, dass** die erste Flüssigkeit (3) der ersten Emulsion (4) gemäß Schritt (b) derart gewählt wird, dass die Löslichkeit zumindest der wenigstens einen ersten amphiphilen Verbindung (2) in der ersten Flüssigkeit (3), insbesondere die Löslichkeit sowohl der wenigstens einen ersten amphiphilen Verbindung (2) als auch der wenigstens einen zweiten amphiphilen Verbindung (8) in der ersten Flüssigkeit (3), höchstens 1 × 10⁻⁵ mol/l beträgt.

4. Verfahren nach einem der Ansprüche 1 bis 3, **dadurch gekennzeichnet, dass** die erste Flüssigkeit (3) der ersten Emulsion (4) gemäß Schritt (b) derart gewählt wird, dass die Löslichkeit zumindest der wenigstens einen ersten amphiphilen Verbindung (2), insbesondere sowohl der wenigstens einen ersten amphiphilen Verbindung (2) als auch der wenigstens einen zweiten amphiphilen Verbindung (8), in der ersten Flüssigkeit (3) höchstens 0,5 x 10⁻⁵ mol/l, insbesondere höchstens 1 × 10⁻⁶ mol/l, vorzugsweise höchstens 1 × 10⁻⁷ mol/l, beträgt.

5. Verfahren nach einem der Ansprüche 2 bis 4, **dadurch gekennzeichnet, dass** die mit der, insbesondere hydrophilen, flüssigen Phase der zweiten Emulsion (9) nicht oder schlecht mischbare, insbesondere hydrophobe, zweite Flüssigkeit (10) entsprechend der, insbesondere hydrophoben, ersten Flüssigkeit (3) der ersten Emulsion (4) gemäß Schritt (b) gewählt wird.

6. Verfahren nach einem der Ansprüche 2 bis 5, **dadurch gekennzeichnet, dass** die Mischung (7) in Form der zweiten Emulsion (9) gemäß Schritt (c) dadurch erzeugt wird, indem
- zunächst eine Mischung aus der flüssigen Phase gemäß Schritt (c) und der wenigstens einen zweiten amphiphilen Verbindung (8) bereitgestellt wird; wonach
- die zweite Flüssigkeit (10) in dieser Mischung unter Bildung der zweiten Emulsion (9) emulgiert wird, indem die zweite Flüssigkeit (10) in die Mischung eindispergiert wird.

7. Verfahren nach einem der Ansprüche 1 bis 6, **dadurch gekennzeichnet, dass** die erste Emulsion (4) gemäß Schritt (b) dadurch erzeugt wird, indem zunächst
- eine Mischung aus der Wirkstofflösung (1) des zu verkapselnden Wirkstoffes gemäß Schritt (a) und der wenigstens einen ersten amphiphilen Verbindung (2) bereitgestellt wird; wonach
- diese Mischung unter Bildung der ersten Emulsion (4) gemäß Schritt (b) in der ersten Flüssigkeit (3) emulgiert wird, indem die Mischung in die erste Flüssigkeit (3) eindispergiert wird.

8. Verfahren nach einem der Ansprüche 1 bis 7, **dadurch gekennzeichnet, dass**
- als erste Flüssigkeit (3) der ersten Emulsion (4) gemäß Schritt (b) eine hydrophobe Flüssigkeit eingesetzt wird, welche insbesondere aus der Gruppe der flüssigen halogenierten Kohlenwasserstoffe einschließlich der Fluorcarbone, der Silikonöle und der Siloxane einschließlich Mischungen hiervon gewählt wird; und/oder
- als Lösungsmittel der Wirkstofflösung (1) gemäß Schritt (a) ein hydrophiles Lösungsmittel, insbesondere auf wässriger und/oder alkoholischer Basis, eingesetzt wird.

9. Verfahren nach einem der Ansprüche 1 bis 8, **dadurch gekennzeichnet, dass**
- die erste Flüssigkeit (3) der ersten Emulsion (4) mit den hierin emulgierten Tropfen der Wirkstofflösung (1) mit der hieran angelagerten, zumindest monomolekularen, inneren Schicht der wenigstens einen ersten amphiphilen Verbindung (2) gemäß Schritt (b) derart gewählt wird, dass sie einen niedrigeren Schmelzpunkt aufweist als die Wirkstofflösung (1);
- die erste Emulsion (4) gemäß Schritt (b) auf eine Temperatur zwischen dem Schmelzpunkt der ersten Flüssigkeit (3) der ersten Emulsion (4) und dem Schmelzpunkt der Wirkstofflösung (1) abgekühlt wird, um die in der ersten Flüssigkeit (3) emulgierten Tropfen der Wirkstofflösung (1) mit der an diese angelagerten, zumindest monomolekularen, inneren Schicht der wenigstens einen ersten amphiphilen Verbindung (2) gemäß Schritt (b) in den festen Zustand zu überführen; und sodann
- die erste Emulsion (4) gemäß Schritt (b) im festen Zustand der Wirkstofflösung (4) mit der mit der ersten Flüssigkeit (3) der ersten Emulsion (4) gemäß Schritt (b) nicht oder schlecht mischbaren flüssigen Phase der Mischung (7) bzw. der zweiten Emulsion (9) mit der wenigstens einen zweiten amphiphilen Verbindung (8) gemäß Schritt (c) unter Ausbildung der Phasengrenze (6) gemäß Schritt (d) in Kontakt gebracht wird und die über die Phasengrenze (6) miteinander in Kontakt stehenden erste Emulsion (4) und die Mischung (7) bzw. die zweite Emulsion (9) gemäß Schritt (e) zentrifugiert werden.

10. Verfahren nach Anspruch 9, **dadurch gekennzeichnet, dass** die Wirkstofflösung (1) mit der an diese angelagerten, zumindest monomolekularen, inneren Schicht der wenigstens einen ersten amphiphilen Verbindung (2) während des Zentrifugierens im festen Zustand gehalten wird, um sie aufgrund dadurch bedingten Dichteunterschiedes von der Phasengrenze (6) fort in Richtung der Mischung (7) bzw. der zweiten Emulsion (9) zu bewegen.

11. Verfahren nach einem der Ansprüche 1 bis 10, **dadurch gekennzeichnet, dass** die Bischicht aus den beiden zumindest monomolekularen Schichten der wenigstens einen ersten amphiphilen Verbindung (2) und der wenigstens einen zweiten amphiphilen Verbindung (8), insbesondere ausschließlich die äußere, zumindest monomolekulare Schicht der wenigstens einen zweiten amphiphilen Verbindung (8), durch Reaktion mit hydrophilen Polymerkonjugaten modifiziert wird.

12. Verfahren nach einem der Ansprüche 1 bis 11, **dadurch gekennzeichnet, dass** es
- chargenweise in einer diskontinuierlichen Zentrifugiereinrichtung durchgeführt wird, indem einerseits die erste Emulsion (4) gemäß Schritt (b), andererseits die Mischung (7) bzw. die zweite Emulsion (9) gemäß Schritt (c) der Zentrifugiereinrichtung aufgegeben werden, wonach diese zentrifugiert werden, wonach der Zentrifugiereinrichtung einerseits die flüssige Phase mit den mit der Bischicht aus den beiden zumindest monomolekularen Schichten der wenigstens einen ersten amphiphilen Verbindung (2) und der wenigstens einen zweiten amphiphilen Verbindung (8) versehenen Liposomen (L), andererseits die erste Flüssigkeit (3) entnommen werden; oder
- semikontinuierlich in einer diskontinuierlichen Zentrifugiereinrichtung durchgeführt wird, indem einerseits die erste Emulsion (4) gemäß Schritt (b), andererseits die Mischung (7) bzw. die zweite Emulsion (9) gemäß Schritt (c) der Zentrifugiereinrichtung über einen Zeitraum kontinuierlich aufgegeben und diese währenddessen zentrifugiert werden, wonach der Zentrifugiereinrichtung einerseits die flüssige Phase mit den mit der Bischicht aus den beiden zumindest monomolekularen Schichten der wenigstens einen ersten amphiphilen Verbindung (2) und der wenigstens einen zweiten amphiphilen Verbindung (8) versehenen Liposomen (L), andererseits die erste Flüssigkeit (3) entnommen werden.

13. Verfahren nach einem der Ansprüche 1 bis 11, **dadurch gekennzeichnet, dass** es kontinuierlich in einer im Durchfluss betriebenen, kontinuierlichen Zentrifugiereinrichtung durchgeführt wird, indem einerseits die erste Emulsion (4) gemäß Schritt (b), andererseits die Mischung (7) bzw. die zweite Emulsion (9) gemäß Schritt (c) der Zentrifugiereinrichtung kontinuierlich aufgegeben und zentrifugiert und der Zentrifugiereinrichtung einerseits die flüssige Phase mit den mit der Bischicht aus den beiden zumindest monomolekularen Schichten der wenigstens einen ersten amphiphilen Verbindung (2) und der wenigstens einen zweiten amphiphilen Verbindung (8) versehenen Liposomen (L), andererseits die erste Flüssigkeit (3) kontinuierlich entnommen werden.

14. Verfahren nach Anspruch 13, **dadurch gekennzeichnet, dass**
- einerseits die erste Emulsion (4) gemäß Schritt (b), andererseits die Mischung (7) bzw. die zweite Emulsion (9) gemäß Schritt (c) der kontinuierlichen Zentrifugiereinrichtung über separate Einlässe (14, 15) aufgegeben und in einem Einlassbereich der Zentrifugiereinrichtung mittels eines sich im Wesentlichen in Radialrichtung derselben erstreckenden Einlasswehres (16) voneinander getrennt gehalten werden, wonach sie infolge Passierens des Einlasswehres (16) in eine gemeinsame Zentrifugierkammer (13) überführt werden; und/oder
- einerseits die flüssige Phase mit den mit der Bischicht aus den beiden zumindest monomolekularen Schichten der wenigstens einen ersten amphiphilen Verbindung (2) und der wenigstens einen zweiten amphiphilen Verbindung (8) versehenen Liposomen (L), andererseits die erste Flüssigkeit (3) aus einer gemeinsamen Zentrifugierkammer (13) der Zentrifugiereinrichtung mittels eines sich im Wesentlichen in Radialrichtung derselben erstreckenden Auslasswehres (19) voneinander getrennt und der Zentrifugiereinrichtung über separate Auslässe (17, 18) entnommen werden.

15. Verfahren nach Anspruch 13 oder 14, **dadurch gekennzeichnet, dass**
- die in der ersten Flüssigkeit (3) emulgierten Tropfen der Wirkstofflösung (1) mit der an diese angelagerten, zumindest monomolekularen, inneren Schicht der wenigstens einen ersten amphiphilen Verbindung (2) der ersten Emulsion (4) gemäß Schritt (b) in einer Zentrifugierkammer (13) der kontinuierlichen Zentrifugiereinrichtung mittels eines in der Zentrifugierkammer (13), insbesondere in deren auslassseitigen Abschnitt, angeordneten und sich im Wesentlichen in Radialrichtung derselben erstreckenden ersten Rückhaltewehres (20) akkumuliert werden; und/oder
- die in der flüssigen Phase der zweiten Emulsion (9) emulgierten Tropfen der zweiten Flüssigkeit (10) mit der hieran angelagerten, zumindest monomolekularen Schicht der wenigstens einen zweiten amphiphilen Verbindung (8) in einer Zentrifugierkammer (13) der kontinuierlichen Zentrifugiereinrichtung mittels eines in der Zentrifugierkammer (13), insbesondere in deren auslassseitigen Abschnitt, angeordneten und sich im Wesentlichen in Radialrichtung derselben erstreckenden zweiten Rückhaltewehres (21) akkumuliert werden.

## Claims

1. A method of encapsulating active ingredients in liposomes (L), comprising
- a solution (1), especially a hydrophilic solution, of the active ingredient and
- at least one bilayer composed of two at least monomolecular layers of at least one first amphiphilic compound (2) and at least one second amphiphilic compound (8), especially each from the group of the lipids,
wherein the active ingredient solution (1) is encapsulated by the bilayer, comprising the following steps:
(a) providing an active ingredient solution (1), especially a hydrophilic active ingredient solution, of the active ingredient to be encapsulated by dissolving the active ingredient in at least one solvent, especially a hydrophilic solvent;
(b) providing a first emulsion (4) by emulsifying the active ingredient solution (1) from step (a) in at least one first liquid (3), especially a hydrophobic first liquid, having zero or sparing miscibility with the at least one solvent of the active ingredient solution (1), in the presence of the at least one first amphiphilic compound (2), in order to accumulate an at least monomolecular inner layer of the at least one first amphiphilic compound (2) on the droplets of the active ingredient solution (1) emulsified in the first liquid (3);
(c) providing a mixture (7) of a liquid phase, especially a hydrophilic liquid phase, having zero or sparing miscibility with the first liquid (3) of the first emulsion (4) from step (b) with the at least one second amphiphilic compound (8);
(d) contacting the first emulsion (4) composed of the first liquid (3) and having the droplets of the active ingredient solution (1) that are emulsified therein and having the at least monomolecular inner layer of the at least one first amphiphilic compound (2) that has been accumulated thereon from step (b) with the mixture (7) of the liquid phase with the at least one second amphiphilic compound (8) from step (c) to form a phase boundary (6) between the first emulsion (4) from step (b) and the mixture (7) from step (c), wherein the at least one second amphiphilic compound (8) is enriched at the phase boundary (6); and
(e) centrifuging the first emulsion (4) from step (b) and the mixture (7) from step (c) that are in contact with one another via the phase boundary (6), in order to transfer the droplets of the active ingredient solution (1) present in the first emulsion (4) and having the at least monomolecular inner layer of the at least one first amphiphilic compound (2) that has been accumulated thereon from the first liquid (3) of the first emulsion (4) from step (b) into the liquid phase of the mixture (7) from step (c), wherein, when it passes the phase boundary (6), the at least one second amphiphilic compound (8) enriched there is accumulated on the at least monomolecular inner layer of the at least one first amphiphilic compound (2) of the droplets of the active ingredient solution (1) to form an at least monomolecular outer layer thereof, in order to produce the bilayer composed of the two at least monomolecular layers of the at least one first amphiphilic compound (2) and the at least one second amphiphilic compound (8).

2. The method as claimed in claim 1, **characterized in that**
- the mixture (7) of the liquid phase, especially the hydrophilic liquid phase, with the at least one second amphiphilic compound (8) used in step (c) is a second emulsion (9) of the liquid phase and at least one second liquid (10), especially a hydrophobic second liquid, having zero or sparing miscibility therewith with the at least one second amphiphilic compound (8), **in that** the second liquid (10) is emulsified in the liquid phase in the presence of the at least one second amphiphilic compound (8) in order to accumulate an at least monomolecular layer of the at least one second amphiphilic compound (8) on the droplets of the second liquid (10) emulsified in the liquid phase and to immobilize the second amphiphilic compound (8) on these droplets in this way; and
- on centrifugation of the first emulsion (4) from step (b) and the mixture (7) in the form of the second emulsion (9) from step (c) that are in contact with one another via the phase boundary (6), the droplets of the second liquid (10) with the at least monomolecular layer of the at least one second amphiphilic compound (8) accumulated thereon are constantly transferred from the liquid phase of the second emulsion (9) to the phase boundary (6) between the first emulsion (4) and the second emulsion (9), in order to constantly enrich the at least one second amphiphilic compound (8) at the phase boundary (6).

3. The method as claimed in claim 1 or 2, **characterized in that** the first liquid (3) of the first emulsion (4) from step (b) is chosen such that the solubility at least of the at least one first amphiphilic compound (2), in particular both of the at least one first amphiphilic compound (2) and of the at least one second amphiphilic compound (8), in the first liquid (3) is not more than 1 × 10⁻⁵ mol/l.

4. The method as claimed in any of claims 1 to 3, **characterized in that** the first liquid (3) of the first emulsion (4) from step (b) is chosen such that the solubility at least of the at least one first amphiphilic compound (2), especially both of the at least one first amphiphilic compound (2) and of the at least one second amphiphilic compound (8), in the first liquid (3) is not more than 0.5 x 10⁻⁵ mol/l, especially not more than 1 × 10⁻⁶ mol/l, preferably not more than 1 × 10⁻⁷ mol/l.

5. The method as claimed in any of claims 2 to 4, **characterized in that** the second liquid (10), especially the hydrophobic second liquid, having zero or sparing miscibility with the liquid phase, especially the hydrophilic liquid phase, of the second emulsion (9) is chosen to correspond to the first liquid (3), especially hydrophobic first liquid, of the first emulsion (4) from step (b).

6. The method as claimed in any of claims 2 to 5, **characterized in that** the mixture (7) in the form of the second emulsion (9) from step (c) is created by
- first providing a mixture of the liquid phase from step (c) and the at least one second amphiphilic compound (8); and then
- emulsifying the second liquid (10) in this mixture to form the second emulsion (9), by dispersing the second liquid (10) into the mixture.

7. The method as claimed in any of claims 1 to 6, **characterized in that** the first emulsion (4) from step (b) is created by first
- providing a mixture of the active ingredient solution (1) of the active ingredient to be encapsulated from step (a) and the at least one first amphiphilic compound (2); and then
- emulsifying this mixture to form the first emulsion (4) from step (b) in the first liquid (3) by dispersing the mixture into the first liquid (3).

8. The method as claimed in any of claims 1 to 7, **characterized in that**
- the first liquid (3) used in the first emulsion (4) from step (b) is a hydrophobic liquid which is especially selected from the group of the liquid halogenated hydrocarbons, including the fluorocarbons, silicone oils and siloxanes including mixtures thereof; and/or
- the solvent used in the active ingredient solution (1) from step (a) is a hydrophilic solvent, especially a water- and/or alcohol-based solvent.

9. The method as claimed in any of claims 1 to 8, **characterized in that**
- the first liquid (3) of the first emulsion (4) with the droplets of the active ingredient solution (1) emulsified therein and having the at least monomolecular inner layer of the at least one first amphiphilic compound (2) accumulated thereon from step (b) is chosen such that it has a lower melting point than the active ingredient solution (1);
- the first emulsion (4) from step (b) is cooled down to a temperature between the melting point of the first liquid (3) of the first emulsion (4) and the melting point of the active ingredient solution (1), in order to convert the droplets of the active ingredient solution (1) emulsified in the first liquid (3) and having the at least monomolecular inner layer of the at least one first amphiphilic compound (2) accumulated thereon from step (b) to the solid state; and then
- the first emulsion (4) from step (b) in the solid state of the active ingredient solution (4) is contacted with the liquid phase of the mixture (7), or of the second emulsion (9) with the at least one second amphiphilic compound (8) from step (c), that has zero or sparing miscibility with the first liquid (3) of the first emulsion (4) from step (b) to form the phase boundary (6) according to step (d), and the first emulsion (4) and the mixture (7) or second emulsion (9) that are in contact with one another via the phase boundary (6) from step (e) are centrifuged.

10. The method as claimed in claim 9, **characterized in that** the active ingredient solution (1) having the at least monomolecular inner layer of the at least one first amphiphilic compound (2) accumulated thereon is kept in the solid state during the centrifuging, in order to move it, on account of a resultant difference in density, onward from the phase boundary (6) in the direction of the mixture (7) or of the second emulsion (9) .

11. The method as claimed in any of claims 1 to 10, **characterized in that** the bilayer composed of the two at least monomolecular layers of the at least one first amphiphilic compound (2) and of the at least one second amphiphilic compound (8), especially exclusively the outer at least monomolecular layer of the at least one second amphiphilic compound (8), is modified by reaction with hydrophilic polymer conjugates.

12. The method as claimed in any of claims 1 to 11, **characterized in that** it is performed
- batchwise in a batchwise centrifuge device by firstly introducing the first emulsion (4) from step (b), and secondly the mixture (7) or second emulsion (9) from step (c), into the centrifuge device, and then centrifuging them, and then firstly the liquid phase comprising the liposomes (L) provided with the bilayer of the two at least monomolecular layers of the at least one first amphiphilic compound (2) and the at least one second amphiphilic compound (8), and secondly the first liquid (3), are withdrawn from the centrifuge device; or
- semicontinuously in a batchwise centrifuge device by continuously introducing firstly the first emulsion (4) from step (b), and secondly the mixture (7) or second emulsion (9) from step (c), into the centrifuge device over a period of time, during which they are centrifuged, and then firstly the liquid phase comprising the liposomes (L) provided with the bilayer of the two at least monomolecular layers of the at least one first amphiphilic compound (2) and the at least one second amphiphilic compound (8), and secondly the first liquid (3), are withdrawn from the centrifuge device.

13. The method as claimed in any of claims 1 to 11, **characterized in that** it is performed continuously in a flow-operated continuous centrifuge device by continuously introducing firstly the first emulsion (4) from step (b), and secondly the mixture (7) or second emulsion (9) from step (c), into the centrifugation device, and centrifuging and continuously withdrawing firstly the liquid phase comprising the liposomes (L) provided with the bilayer of the two at least monomolecular layers of the at least one first amphiphilic compound (2) and the at least one second amphiphilic compound (8), and secondly the first liquid (3), from the centrifuge device.

14. The method as claimed in claim 13, **characterized in that**
- firstly the first emulsion (4) from step (b), and secondly the mixture (7) or second emulsion (9) from step (c), are introduced into the continuous centrifuge device via separate inlets (14, 15) and kept separate from one another in an intake region of the centrifuge device by means of an inlet weir (16) that extends essentially in radial direction thereof, after which, as a result of passage through the inlet weir (16), they are transferred into a common centrifuge chamber (13);
and/or
- firstly the liquid phase comprising the liposomes (L) provided with the bilayer of the two at least monomolecular layers of the at least one amphiphilic compound (2) and the at least one second amphiphilic compound (8), and secondly the first liquid (3), from a common centrifuge chamber (13) of the centrifuge device are separated from one another by means of an outlet weir (19) that extends essentially in radial direction thereof, and withdrawn from the centrifuge device via separate outlets (17, 18).

15. The method as claimed in claim 13 or 14, **characterized in that**
- the droplets of the active ingredient solution (1) with the at least monomolecular inner layer of the at least one first amphiphilic compound (2) of the first emulsion (4) accumulated thereon that are emulsified in the first liquid (3) from step (b) are accumulated in a centrifuge chamber (13) of the continuous centrifuge device by means of a first retaining weir (20) which is disposed in the centrifuge chamber (13), especially in the outlet-side section thereof, and extends essentially in radial direction thereof; and/or
- the droplets of the second liquid (10) with the at least monomolecular layer of the at least one second amphiphilic compound (8) accumulated thereon that are emulsified in the liquid phase of the second emulsion (9) are accumulated in a centrifuge chamber (13) of the continuous centrifuge device by means of a second retaining weir (21) which is disposed in the centrifuge chamber (13), especially in the outlet-side section thereof, and extends essentially in radial direction thereof.

## Revendications

1. Procédé pour encapsuler des principes actifs dans des liposomes (L), comprenant :
- une solution (1), notamment hydrophile, du principe actif ; et
- au moins une bicouche composée de deux couches au moins mononucléaires comprenant au moins une première liaison amphiphile (2) et au moins une deuxième liaison amphiphile (8), notamment respectivement appartenant au groupe des lipides ;
l'encapsulement de la solution de principe actif (1) par la bicouche comprenant les étapes suivantes :
(a) mise à disposition d'une solution de principe actif (1), notamment hygrophile, du principe actif à encapsuler, en dissolvant le principe actif dans au moins un solvant, notamment hydrophile ;
(b) mise à disposition d'une première émulsion (4) par émulsification de la solution de principe actif (1) selon l'étape (a) dans au moins un premier fluide (3), notamment hydrophobe, non miscible, ou seulement difficilement, avec l'au moins un solvant de la solution de principe actif (1), en présence de l'au moins une première liaison amphiphile (2), pour fixer une couche intérieure au moins mononucléaire de l'au moins une première liaison amphiphile (2) au niveau des gouttes de la solution de principe actif (1) émulsifiées dans le premier fluide (3) ;
(c) mise à disposition d'un mélange (7) composé d'une phase fluide, notamment hydrophile, non miscible, ou seulement difficilement, avec le premier fluide (3) de la première émulsion (4) selon l'étape (b), avec l'au moins une deuxième liaison amphiphile (8) ;
(d) mise en contact de la première émulsion (4) composée du premier fluide (3) avec les gouttes de la solution de principe actif (1) émulsifiées dedans avec la couche intérieure au moins mononucléaire disposée contre de l'au moins une première liaison amphiphile (2) selon l'étape (b) avec le mélange (7) de la phase fluide avec l'au moins une deuxième liaison amphiphile (8) selon l'étape (c) par formation d'une limite de phase (6) entre la première émulsion (4) selon l'étape (b) et le mélange (7) selon l'étape (c), l'au moins une deuxième liaison amphiphile (8) étant enrichie au niveau de la limite de phase (6) ; et
(e) la centrifugation de la première émulsion (4) selon l'étape (b) et du mélange (7) selon l'étape (c) en contact entre eux via la limite de phase (6), pour transformer les gouttes de la solution de principe actif (1) contenue dans la première émulsion (4) avec la couche intérieure au moins mononucléaire y étant fixée de l'au moins une première liaison amphiphile (2) à partir du premier fluide (3) de la première émulsion (4) selon l'étape (b) jusque dans la phase fluide du mélange (7) selon l'étape (c), sachant que lors du passage de la limite de phase (6), l'au moins une deuxième liaison amphiphile (8) y étant enrichie est fixée par formation d'une couche extérieure au moins mononucléaire de celle-ci à la couche intérieure au moins mononucléaire de l'au moins une première liaison amphiphile (2) des gouttes de la solution de principe actif (1), pour produire la bicouche composée des deux couches au moins mononucléaires de l'au moins une première liaison amphiphile (2) et l'au moins une deuxième liaison amphiphile (8).

2. Procédé selon la revendication 1, **caractérisé en ce que** :
- le mélange (7) composé de la phase fluide, notamment hydrophile, avec l'au moins une deuxième liaison amphiphile (8) selon l'étape (c) utilisé est une deuxième émulsion (9) composée de la phase fluide et d'au moins un deuxième fluide (10), notamment hydrophobe, non miscible avec lui, ou seulement difficilement, avec l'au moins une deuxième liaison amphiphile (8), le deuxième fluide (10) étant émulsifié dans la phase fluide en présence d'au moins une deuxième liaison amphiphile (8), pour fixer une couche au moins mononucléaire de l'au moins une deuxième liaison amphiphile (8) au niveau des gouttes du deuxième fluide (10) émulsifiées dans la phase fluide et pour immobiliser la deuxième liaison amphiphile (8) de cette façon au niveau de ces gouttes ; et
- lors de la centrifugation de de la première émulsion (4) selon l'étape (b) et du mélange (7) selon l'étape (c) présent sous la forme de la deuxième émulsion (9) en contact entre eux via la limite de phase (6), les gouttes du deuxième fluide (10) avec la couche au moins mononucléaire y étant fixée de l'au moins une deuxième liaison amphiphile (8) est transférée de la phase fluide de la deuxième émulsion (9) en continu au niveau de la limite de phase (6) entre la première émulsion (4) et la deuxième émulsion (9), pour enrichir l'au moins une deuxième liaison amphiphile (8) en continu au niveau de la limite de phase (6).

3. Procédé selon la revendication 1 ou 2, **caractérisé en ce que** le premier fluide (3) de la première émulsion (4) selon l'étape (b) est sélectionné de telle sorte que la solubilité au moins de l'au moins une première liaison amphiphile (2) dans le premier fluide (3), notamment la solubilité tant de l'au moins une première liaison amphiphile (2) que de l'au moins une deuxième liaison amphiphile (8) dans le premier fluide (3) soit de tout au plus 1 × 10⁻⁵ mol/l.

4. Procédé selon l'une quelconque des revendications 1 à 3, **caractérisé en ce que** le premier fluide (3) de la première émulsion (4) selon l'étape (b) est sélectionné de telle sorte que la solubilité au moins de l'au moins une première liaison amphiphile (2), notamment tant de l'au moins une première liaison amphiphile (2) que de l'au moins une deuxième liaison amphiphile (8), soit de tout au plus 0,5 x 10⁻⁵ mol/l dans le premier fluide (3), notamment de tout au plus 1 × 10⁻⁶ mol/l, de préférence de tout au plus 1 × 10⁻⁷ mol/l.

5. Procédé selon l'une quelconque des revendications 2 à 4, **caractérisé en ce que** le deuxième fluide (10), notamment hydrophobe, non miscible, ou seulement difficilement, avec la phase fluide, notamment hydrophile, de la deuxième émulsion (9) soit sélectionné en fonction du premier fluide (3), notamment hydrophobe, de la première émulsion (4) selon l'étape (b).

6. Procédé selon l'une quelconque des revendications 2 à 5, **caractérisé en ce que** le mélange (7) est produit sous la forme de la deuxième émulsion (9) selon l'étape (c) :
- en mettant d'abord à disposition un mélange composé de la phase fluide selon l'étape (c) et de l'au moins une deuxième liaison amphiphile (8) ; après quoi
- le deuxième fluide (10) contenu dans ce mélange est émulsifié en formant la deuxième émulsion (9) par dispersion du deuxième fluide (10) dans le mélange.

7. Procédé selon l'une quelconque des revendications 1 à 6, **caractérisé en ce que** la première émulsion (4) selon l'étape (b) est produite :
- d'abord en mettant à disposition un mélange composé de la solution de principe actif (1) du principe actif à encapsuler selon l'étape (a) et de l'au moins une première liaison amphiphile (2) ; à la suite de quoi
- ce mélange est émulsifié par formation de la première émulsion (4) selon l'étape (b) dans le premier fluide (3), par dispersion du mélange dans le premier fluide (3).

8. Procédé selon l'une quelconque des revendications 1 à 7, **caractérisé en ce que** :
- le premier fluide (3) de la première émulsion (4) selon l'étape (b) utilisé est un fluide hydrophobe qui est notamment sélectionné dans le groupe des hydrocarbures halogénérés fluides incluant les fluorocarbures, les huiles de silicone et le siloxane y compris des mélanges de ceux-ci ; et/ou
- le solvant de la solution de principe actif (1) selon l'étape (a) utilisé est un solvant hydrophile, notamment à base aqueuse et/ou alcoolisée.

9. Procédé selon l'une quelconque des revendications 1 à 8, **caractérisé en ce que** :
- le premier fluide (3) de la première émulsion (4) avec les gouttes de la solution de principe actif (1) émulsifiées dedans est sélectionné de telle sorte avec la couche intérieure au moins mononucléaire y étant fixée de l'au moins une première liaison amphiphile (2) selon l'étape (b) qu'il comporte un point de fusion plus faible que la solution de principe actif (1) ;
- la première émulsion (4) selon l'étape (b) est refroidie à une température comprise entre le point de fusion du premier fluide (3) de la première émulsion (4) et le point de fusion de la solution de principe actif (1) pour transférer les gouttes de la solution de principe actif (1) émulsifiées dans le premier fluide (3) avec la couche intérieure au moins mononucléaire y étant fixée de l'au moins une première liaison amphiphile (2) selon l'étape (b) dans l'état solide ; puis
- la première émulsion (4) selon l'étape (b) amenée en contact à l'état solide de la solution de principe actif (4) avec la phase fluide, non miscible, ou seulement difficilement, avec le premier fluide (3), de la première émulsion (4) selon l'étape (b) du mélange (7) et/ou de la deuxième émulsion (9) avec l'au moins une deuxième liaison amphiphile (8) selon l'étape (c) par formation de la limite de phase (6) selon l'étape (d) et que la première émulsion (4) et le mélange (7) en contact entre eux via la limite de phase (6) et/ou la deuxième émulsion (9) selon l'étape (e) sont centrifugés.

10. Procédé selon la revendication 9, **caractérisé en ce que** la solution de principe actif (1) avec la couche intérieure au moins mononucléaire fixée à elle de l'au moins une première liaison amphiphile (2) est maintenue à l'état solide pendant la centrifugation, pour la déplacer sur la base d'une certaine différence de masse volumique de la limite de phase (6) en direction du mélange (7) et/ou de la deuxième émulsion (9).

11. Procédé selon l'une quelconque des revendications 1 à 10, **caractérisé en ce que** la bicouche composée des deux couches au moins mononucléaires de l'au moins une première liaison amphiphile (2) et de l'au moins une deuxième liaison amphiphile (8), notamment uniquement la couche extérieure au moins mononucléaire de l'au moins une deuxième liaison amphiphile (8), est modifiée par réaction avec des polymères conjugués hydrophiles.

12. Procédé selon l'une quelconque des revendications 1 à 11, **caractérisé en ce qu'**il :
- est réalisé à la façon d'une charge dans une installation de centrifugation discontinue en amenant d'une part la première émulsion (4) selon l'étape (b), d'autre part le mélange (7) et/ou la deuxième émulsion (9) selon l'étape (c) à l'installation de centrifugation, après quoi ils sont centrifugés, après quoi on retire de l'installation de centrifugation d'une part la phase fluide avec les liposomes (L) avec la bicouche pourvue des deux couches au moins mononucléaires de l'au moins une première liaison amphiphile (2) et de l'au moins une deuxième liaison amphiphile (8), d'autre part le premier fluide (3) ; ou
- est réalisé en semi-continu dans une installation de centrifugation discontinue en amenant d'une part en continu la première émulsion (4) selon l'étape (b), d'autre part le mélange (7) et/ou la deuxième émulsion (9) selon l'étape (c) à l'installation de centrifugation pendant un certain laps de temps et en les centrifugeant pendant ce temps, après quoi on retire de l'installation de centrifugation d'une part la phase fluide avec les liposomes (L) pourvus de la bicouche composée de deux couches au moins mononucléaires de l'au moins une première liaison amphiphile (2) et de l'au moins un deuxième liaison amphiphile (8), d'autre part le premier fluide (3).

13. Procédé selon l'une quelconque des revendications 1 à 11, **caractérisé en ce qu'**il est réalisé en continu dans une installation de centrifugation continue entraînée dans le débit en amenant d'une part en continu la première émulsion (4) selon l'étape (b), d'autre part le mélange (7) et/ou la deuxième émulsion (9) selon l'étape (c) à l'installation de centrifugation et en les centrifugeant et en retirant en continu de l'installation de centrifugation d'une part la phase fluide avec les liposomes (L) pourvus de la bicouche composée des deux couches au moins mononucléaires de l'au moins une première liaison amphiphile (2) et de l'au moins une deuxième liaison amphiphile (8), d'autre part le premier fluide (3).

14. Procédé selon la revendication 13, **caractérisé en ce que** :
- d'une part la première émulsion (4) selon l'étape (b), d'autre part le mélange (7) et/ou la deuxième émulsion (9) selon l'étape (c), sont amenés en continu à l'installation de centrifugation via des admissions (14, 15) séparées et sont maintenus séparément les uns des autres dans une zone d'admission de l'installation de centrifugation à l'aide d'un déversoir d'admission (16) s'étendant pour l'essentiel dans la direction radiale de celle-ci, après quoi ils sont transférés suite au passage du déversoir d'admission (16) jusque dans une chambre de centrifugation (13) commune ; et/ou
- d'une part la phase fluide avec les liposomes (L) pourvus de la bicouche composée des deux couches au moins mononucléaires de l'au moins une première liaison amphiphile (2) et de l'au moins une deuxième liaison amphiphile (8), d'autre part le premier fluide (3), sont séparés l'un de l'autre au sortir de la chambre de centrifugation (13) commune de l'installation de centrifugation à l'aide d'un déversoir de sortie (19) s'étendant pour l'essentiel dans la direction radiale de celle-ci et sont retirés de l'installation de centrifugation via des sorties (17, 18) séparées.

15. Procédé selon la revendication 13 ou 14, **caractérisé en ce que** :
- les gouttes de la solution de principe actif (1) émulsifiées dans le premier fluide (3) sont accumulées avec la couche intérieure au moins mononucléaire fixée à elle de l'au moins une première liaison amphiphile (2) de la première émulsion (4) selon l'étape (b) dans une chambre de centrifugation (13) de l'installation de centrifugation continue à l'aide d'un premier déversoir de retenue (20) disposé dans la chambre de centrifugation (13), notamment dans sa section située du côté de sortie et s'étendant pour l'essentiel dans la direction radiale de celle-ci ; et/ou
- les gouttes du deuxième fluide (10) émulsifiées dans la phase fluide de la deuxième émulsion (9) sont accumulées avec la couche au moins mononucléaire y étant fixée de l'au moins une deuxième liaison amphiphile (8) dans une chambre de centrifugation (13) de l'installation de centrifugation continue à l'aide d'un deuxième déversoir de retenue (21) s'étendant dans la chambre de centrifugation (13), notamment dans sa section située du côté de sortie et s'étendant pour l'essentiel dans la direction radiale de celle-ci.
